# EUROPEAN PATENT SPECIFICATION

(11) **EP 1 360 207 B1**
(45) Date of publication and mention of the grant of the patent: **22.06.2011**
(21) Application number: 01990535.5
(22) Date of filing: 03.12.2001
(51) Int. Cl.: C07K 16/00

(54) **Protein arrays of camelid heavy-chain immunoglobulin variable domains**
Proteinraster aus variablen Domänen der schweren Immunoglobulinkette von Kamelen
Matrice de protéines de domaines d'iimunoglobulines variables de camélidés

(30) Priority: 13.12.2000 EP 00311142
(43) Date of publication of application: 12.11.2003
(73) Proprietor: BAC IP B.V., 1411 GP Naarden (NL)
(72) Inventor: DE HAARD, J. J. W., Unilever Research Vlaardingen, NL-3133 AT Vlaardingen (NL); HERMANS, Pim, Unilever Research Vlaardingen, NL-3133 AT Vlaardingen (NL); LANDA, Ilse, Unilever Research Vlaardingen, NL-3133 AT Vlaardingen (NL); VERRIPS, Cornelis T., NL-3994 ZP Houten (NL)
(74) Representative: Swinkels, Bart Willem
(86) International application number: PCT/EP2001/014471
(87) International publication number: WO 2002/048193

(56) References cited:
- WO-A-00/04389
- WO-A-01/40310
- WO-A-01/44301
- WO-A-99/39210
- WO-A-99/64595
- BUSSOW K: "Proteins in gels, computers, crystals and camels" TRENDS IN BIOTECHNOLOGY, ELSEVIER PUBLICATIONS, CAMBRIDGE, GB, vol. 19, no. 9, 1 September 2001 (2001-09-01), pages 328-329, XP004300181 ISSN: 0167-7799
- MUYLDERMANS S ET AL: "Recognition of antigens by single-domain antibody fragments: the superfluous luxury of paired domains" TIBS TRENDS IN BIOCHEMICAL SCIENCES, ELSEVIER PUBLICATION, CAMBRIDGE, EN, vol. 26, no. 4, 1 April 2001 (2001-04-01), pages 230-235, XP004241851 ISSN: 0968-0004
- ARBABI GHAHROUDI M ET AL: "Selection and identification of single domain antibody fragments from camel heavy-chain antibodies" FEBS LETTERS, ELSEVIER SCIENCE PUBLISHERS, AMSTERDAM, NL, vol. 414, no. 3, 15 September 1997 (1997-09-15), pages 521-526, XP004261105 ISSN: 0014-5793
- CAHILL D J: "Protein and antibody arrays and their medical applications" JOURNAL OF IMMUNOLOGICAL METHODS, ELSEVIER SCIENCE PUBLISHERS B.V.,AMSTERDAM, NL, vol. 250, no. 1-2, 1 April 2001 (2001-04-01), pages 81-91, XP004230696 ISSN: 0022-1759
- HAMERS-CASTERMAN C ET AL: "NATURALLY OCCURRING ANTIBODIES DEVOID OF LIGHT CHAINS" NATURE, MACMILLAN JOURNALS LTD. LONDON, GB, vol. 363, 3 June 1993 (1993-06-03), pages 446-448, XP002048619 ISSN: 0028-0836
- MUYLDERMANS S: "SINGLE DOMAIN CAMEL ANTIBODIES: CURRENT STATUS" REVIEWS IN MOLECULAR BIOTECHNOLOGY, ELSEVIER, AMSTERDAM,, NL, vol. 74, no. 4, June 2001 (2001-06), pages 277-302, XP001057480 ISSN: 1389-0352
- HOOGENBOOM H R: "Designing and optimizing library selection strategies for generating high-affinity antibodies" TRENDS IN BIOTECHNOLOGY, ELSEVIER PUBLICATIONS, CAMBRIDGE, GB, vol. 15, no. 2, 1 February 1997 (1997-02-01), pages 62-70, XP004034115 ISSN: 0167-7799
- HOOGENBOOM H R ET AL: "Antibody phage display technology and its applications" IMMUNOTECHNOLOGY, ELSEVIER SCIENCE PUBLISHERS BV, NL, vol. 4, no. 1, 1 June 1998 (1998-06-01), pages 1-20, XP004127382 ISSN: 1380-2933

## Description

### Field of the Invention

The present invention relates to protein arrays comprising a plurality of antibodies or fragments thereof, the construction of such arrays, and to methods of using them, for example in the parallel detection and analysis of up to a large number of proteins in a sample.

### Background of the Invention

Much recent research in molecular cell biology is moving away from the traditional, "reductionist" approach of isolating and characterising individual structures or molecules from a cell, towards a more holistic approach, in which the diverse metabolic functions of the cell as a whole are related to the sum of the molecules present within (Lander & Weinberg, Science 287, 1777-1782 (2000); Lockhart & Winzeler, Nature 405, 827-836 (2000); Hughes et al, Cell 102, 109-126 (2000)). It is becoming increasingly clear, for example, that progress in understanding and, where appropriate, diagnosing, managing or treating a whole range of conditions involving changes in the metabolism of cells in a living organism benefits from the application of methods that provide as complete a picture as possible of the underlying changes such as the pattern of gene expression and of protein content in the various compartments of the cell. This is true in part because most of the genes or proteins might be hitherto unidentified, so that there would be nothing in existing knowledge to suggest investigating their relevance in the context of the condition of interest. In addition, the condition will, in all but the simplest cases, depend on the composite effect of multiple underlying changes, so that an oversimplified view or, in practical applications, an inaccurate diagnosis or an ineffective treatment could result from focusing only on individual contributory factors.

In part, progress in this area is being driven by the rapid advances in genome sequencing, which have led to the identification of unprecedented numbers of genes and gene products, whose existence and role in the cell have often not previously been recognised (Goffeau et al, Science 274, 562-567 (1996); The C. elegans Sequencing Consortium, Science, 282, 2012-2018 (1998); Venter et al., Science 291, 1304-1351 (2001); Int Human Genome Sequencing Consortium, Nature 409, 860-921 (2001)). One approach to identifying which of these proteins may be important in relation to a particular aspect of cell biology is to compare patterns of expression of the genes encoding them in different types of cell, or under different conditions. To this end, there has been considerable progress in the development of methods for detecting the overall expression profile of a cell, rather than focusing on individual genes, as had typically been done in the past. Key to this has been the development of DNA arrays, in which large numbers of DNA sequences are immobilised in a well defined array on a solid support. These arrays make it possible to characterise the amounts of gene specific mRNAs present in a cell under a given set of physiological conditions, by detecting hybridisation of individual mRNA species to their corresponding DNA fragments in the array. The resulting expression profiles provide a very powerful way of developing new insights into cellular responses to various conditions. For example, this approach has been used to detect changes in the expression of multiple genes during tumorgenesis or ageing (Alizadeh et al., Nature 403, 503-511 (2000); Ly et al., Science 287, 2486-2492 (2000)).

However, changes in mRNA levels do not, in general, correlate directly with changes in levels of the proteins they encode, because of differences in translation rates and stability between different mRNAs, as well as different turnover rates of the proteins (Pandey & Mann, Nature 405, 837-846 (2000); Wilkins et al, 'Proteome Research: Frontiers in Functional Genomics' 1-243 (Springer, Berlin, 1997)). Since it are the proteins that actually facilitate and control the vast majority of the processes taking place in the cell, it is clearly important to be able to supplement gene expression profiling methods with companion methods that directly monitor differences in protein content under various physiological conditions, with a degree of sensitivity and speed comparable to those achievable for monitoring mRNA levels using the DNA arrays.

At present the most commonly used method to analyse the distribution of proteins in a cell is 2D gel electrophoresis. However this method provides neither the sensitivity nor the speed of mRNA profiling with DNA arrays. It is also severely limited in resolution, so that even for a simple eukaryotic cell such as *Saccharomyces cerevisiae*, which has only 6220 genes, 2D gels are incomplete, difficult to reproduce and to interpret. Moreover, nearly always abundant proteins mask the presence of less abundant proteins, which are the proteins involved in important regulation processes in the cell (Anderson & Andeson, Electrophoresis 17, 443-453 (1996); Lottspeich, Ang. Chem. Int. Ed. 38, 2476-2492 (1999)).

Another way to approach the problem of analysing the protein content of such complex systems is to generate a library of antibodies with binding specificity for different proteins and to determine which proteins are actually present in a test system by detection of binding to the cognate antibody using a suitable immunoassay. Recently this approach has also been extended to embrace the potential of array technology, with antibodies being immobilised on a surface and binding of suitably labelled antigens to them, which are detected using an optical imaging system. The limitation of these methods has been, however, that it has been very difficult and expensive to generate a sufficiently complete library of antibodies with sufficient binding affinity to be useful. Moreover, the limited stability of antibodies and their intrinsic sticky nature makes it practically impossible to develop reliable arrays based on traditional antibodies (Borrebaeck, Immunology Today 21 8 (2000)) .

The traditional approach to raising antibodies involves immunising an animal with a single, purified immunogen and isolating the immunoglobulins or cloning and selecting antibody-encoding sequences from this animal. This is a slow process, however, and it depends on identification and purification in considerable quantity of all the individual proteins of interest in advance. Thus, it cannot reasonably be expected to be able to provide a sufficient diversity of antibodies. An alternative approach which has recently come into use involves selecting antibodies capable of binding to at least one of the proteins of interest from an extensive, diverse library of antibodies derived synthetically or from an unimmunised source.

A further significant problem in attempting to correlate differences in the behaviour and properties of cells with differences in protein content is that the overwhelming majority of proteins will not, in fact, be present at significantly different levels in any two different cell conditions, so that most of the information in, for example, an antibody array analysis turns out to be irrelevant to the particular condition of interest. The risk is that those cases where there is a significant difference, particularly if the proteins involved are not among the more abundant ones in the cell under either set of conditions, may be missed against the background of the signals from all the other, irrelevant proteins. A related problem is that only 10% of the proteins represent 90% of the mass on proteins in cells (abundant proteins). If labelling is done on total extracts the low abundant proteins will be labelled only with an efficiency of about 10%, which makes their detection almost impossible, in particular when labelled abundant proteins are present.

The potential of protein profiling in improving our understanding of cell biology under diverse physiological conditions is enormous. It is anticipated that this may lead to a much better grasp of key events in the cell cycle, the development of cancers, metabolic diseases and ageing, to name but a few important areas of interest. In turn, this may lead to medical advances, such as the development of new drugs. In order to realise this potential, however, there is a clear need for improved methods of monitoring the levels of a large number of proteins under different conditions and, in some circumstances, for improved methods of focussing on altered levels of proteins, which have been shown to correlate with relevant physiological effects.

WO 00/04389 (Zyomyx) discloses arrays of protein-capture agents, in particular antibodies, for the simultaneous detection of a plurality of proteins which are the expression products, or fragments thereof, of a cell or population of cells in an organism. The arrays are said to be particularly useful for various proteomic applications including assessing patterns of protein expression and modification in cells. According to the description the protein-capture agent, or rather the antibody or antibody fragment, may be derived from a variety of sources, including selection from a library using the phage display method. The antibody or antibody fragments may be derived by a phage display method comprising selection based on binding affinity to the (immobilised) proteins of a cellular extract or a body fluid. Thus, some or many of the antibody fragments of the array would bind proteins of unknown identity and/or function. It is further disclosed that the antibody genes of the phage display libraries may be from immunized donors or, alternatively, the library antibodies may be derived from naive or synthetic libraries. The naive libraries were constructed from spleens of mice which have not been contacted by external antigen.

WO 99/39210 discloses high-density arrays comprising a primary protein array and a secondary antibody array, wherein the secondary array comprises monoclonal antibodies and/or antobody variants or derivatives that bind specifically or non-specifically to one or more proteins in the primary array, and wherein the secondary array is used to determine the protein profile of a cell, tissue, organ or whole organism or a cellular extract, lysate or protein fraction derived therefrom.

Haab et al.; Genome Biology 2(2) 4.1-4.13 (2001) discloses a method for printing microarrays and using these microarrays in a comparative fluorescence assay to measure the abundance of many specific proteins in complex solutions. A robotic device was used to print hundreds of specific hybridoma derived monoclonal antibodies or antigen solutions in an array on the surface of derivatized microscope slides.

Edwards et al., J. Immunological Methods 245 67-78 (2000) describe the isolation and tissue profiles of a large panel of phage antibodies binding to the human adipocyte cell surface.

Hoogenboom et al., Immunotechnology, 4 1-20 (1998) give a review on antibody phage display and its applications.

Muyldermans, Reviews in Molecular Biotechnology 74 277-302 (2001), reports on the current status of single domain camel antibodies.

Frenken et al., J. Biotechnology 78 11-21 (2000) report on the isolation of antigen specific Llama VHH antibody fragments and their high level secretion by *Saccharomyces cerevisiae,* focussing in particular on their binding affinity to haptens.

WO 00/43507 (Unilever) discloses an expression library comprising a repertoire of nucleic acid sequences cloned from an non-immunised source, each encoding at least part of a variable domain of a heavy chain derived from an immunoglobulin naturally devoid of light chains (VHH) wherein the extent of sequence variability in said library is enhanced by introducing mutations in one or more of the complementarity determining regions (CDRs) of said nucleic acid sequences or by generating alternative combinations of CDR and framework sequences not naturally present in the naive library repertoire.

EP-A-0584421 (Casterman et al.) discloses immunoglobulins, or fragments thereof, capable of exhibiting the functional properties of conventional (four-chain) immunoglobulins but which comprise two heavy polypeptide chains and which furthermore are devoid of light polypeptide chains. These immunoglobulins (also referred to as "heavy-chain immunoglobulins") which may be isolated from the serum of Camelids do not rely upon the association of heavy and light chain variable domains for the formation of the antigen-binding site but instead the heavy polypeptide chains alone naturally form the complete antigen binding site. They are thus quite distinct from the heavy chains obtained by the degradation of conventional (four-chain) immunoglobulins (which require a light chain partner, forming a complete antigen binding site for optimal antigen binding).

WO 94/25591 (Unilever) discloses methods for the preparation of such heavy chain antibodies, or fragments thereof, on a large scale comprising transforming a mould or yeast with an expressible DNA sequence encoding said antibody or fragment.

EP-A-0368684 (Medical Research Council) discloses the construction of expression libraries comprising a repertoire of nucleic acid sequences each encoding at least part of an immunoglobulin variable domain and the screening of the encoded domains for binding activities. It is stated that repertoires of genes encoding immunoglobulin variable domains are preferably prepared from lymphocytes of animals immunised with an antigen. The preparation of antigen binding activities from single V_{H} domain, the isolation of which is facilitated by immunisation, is exemplified.

Liu and Marks, Anal. Biochem., 286 119-128 (2000)) describe a selection method on protein spots of a blotted 2D-gel using a human naive single-chain Fᵥ phage display library.

### Summary of the Invention

The object of the invention is defined in the claims. Provided are protein arrays capable of detecting even minor changes in the expression of proteins in cell and tissue extracts and having an optimal signal to noise ratio by removing non-informative abundant proteins from said cell or tissue extracts.

This goal is achieved by providing a protein array, which comprises a plurality of antibodies or antibody fragments, characterised in that said plurality of antibodies or antibody fragments is comprised of heavy-chain variable domain antibodies, or antibody fragments, obtainable from *Camelidae.*

Preferably, the protein array comprises antibodies or antibody fragments from a library comprising cloned DNA sequences encoding antibodies, or antibody fragments, where clones are derived from an unimmunised animal of the genus *Camelidae.*

According to another aspect of the disclosure a diagnostic device is provided comprising the protein array of the invention.

Methods are provided for using such an array for detecting the presence of individual proteins in a sample, comparing the distribution of proteins so revealed in different cell types, and identification of proteins that may be of importance in determining the altered properties of cells in disease, ageing or other conditions.

A method is provided to remove abundant proteins from an extract or sample which do not provide useful information on the condition of a cell or tissue in said extract or sample to be investigated, by affinity chromatography using heavy-chain variable domain antibodies, antibody fragments, obtainable from Camelidae.

A method is provided of assaying in parallel for a plurality of different proteins in a sample which are expression products, or post-translationally modified forms of such expression products, or fragments of either of these, of a cell or a population of cells in an organism, comprising:
(a) delivering the sample to a protein array of the invention under conditions suitable for protein binding, wherein each of the proteins being assayed is a binding partner is a binding partner of the antibody or antibody fragment of at least one patch or hole on the array; and
(b) detecting, either directly or indirectly, for the presence or amount of protein bound to each patch or hole of the array.

A method is provided for determining the proteins expression pattern of a cell or a population of cells in an organism, comprising:
(a) delivering a sample containing the expression products, or post-translationally modified forms of such products, or fragments of either of these, to a protein array of the present invention under conditions suitable for protein binding; and
(b) detecting, either directly or indirectly, for the presence or amount of protein bound to each patch or hole of the array.

A method is provided of comparing the protein expression patterns of two cells or population of cells, comprising:
(a) delivering a sample containing the expression products, or post-translationally modified forms of such products, or fragments of either of these, of a first cell or population of cells to a first protein of the invention under conditions suitable for protein binding;
(b) delivering a sample containing the expression products, or post-translationally modified forms of such products, or fragments of either of these, of a second cell or population of cells to a second array, wherein the second array is identical to the first array;
(c) detecting, either directly or indirectly, for the amount of protein bound to each patch or hole on the washed first and second arrays; and
(d) comparing the amounts of protein bound to the patches or holes of the first array to the amounts of protein bound to the corresponding patches or holes of the second array.

In again another aspect a method is provided of evaluating a disease condition in a tissue in an organism, comprising:
(a) contacting a sample comprising the expression products, or post-translationally modified forms of such products, or fragments of either of these, of the cells of the tissue being evaluated with a protein array of the invention under conditions suitable for protein binding, wherein the binding partners of a plurality of protein-capture agents on the array include proteins which are expression products, or post-translationally modified forms of such products, or fragments of either of these, of the cells of the tissue and whose expression levels are indicative of the disease condition; and
(b) detecting, directly or indirectly, for the amount of protein bound to each patch or hole of the array.

Also disclosed are methods to produce the protein arrays.

In another aspect a method is provided for the simultaneous processing of target antigens and evaluation of selection conditions, also referred to as the micro-panning strategy, which comprises using the combination of panning on a microtiter plate and the predictive value of phage-ELISA, carried out simultaneously

These and other aspects will be outlined in the following detailed description, figures and examples.

### Brief Description of the Drawings

- Fig. 1: is a schematic outline of various approaches on how to select subtractive libraries of antibodies according to the invention and how to lable only the interesting (low abundant) proteins for their detection.
- Fig. 2: shows the amino acid sequence of the mouse Ig cross- reactive VHHs.
- Fig. 3: shows the amino acid sequences of the IgG-subclass specific VHHs.
- Fig. 4: shows VHHs spotted on polylysine coated glass slide and incubated with Cy3 labelled total mouse IgG. 1) VHH B5; 2) VHH C4; 3) VHH E7; 4) VHH H2; 5) anti-GST VHH; 6) PBS.

### Definitions

As used herein, the term "antibody" refers to an immunoglobulin which may be derived from natural sources or synthetically produced in part. A "library", in the context of the invention, is a library of antibodies, each encoded by a cloned unique nucleic acid sequence. The term "naive library", as used herein, is meant to indicate a library obtained from well known sources of mRNA encoding antibodies from animals that have not been immunised. An "immune library" is a library according to this definition, wherein the sequences are cloned from an animal that has previously been immunised with a preparation comprising one or more proteins. A "subtractive antibody library", as used herein, is a collection of antibodies in which each antibody is capable of specific binding to a protein that is present in one chosen cell or tissue type, but not to protein present in the reference cell or tissue type (i.e. originally identical cells but phenotypically different; e.g. old vs. young cells).

### Detailed Description of the Invention

It is an unexpected finding that antibody arrays based on selected heavy-chain variable domain antibodies obtainable from *Camelidae,* also referred to as single-domain antibodies or VHHs, are highly specific. (By "selected" we mean under conditions similar to those which will be used lateron in the array, e.g. high salt and/or high temperature). This makes possible or facilitates the cloning of variable domains of the antibody and the subsequent recombinant expression and selection cognate proteins e.g. by displaying the antibody on the surface of phages or lower eukaryotes.

Single-domain antibodies can be functionalized easily at ther Nor C-terminal end without impairing their functionality, stability of produceability. The fact that only VHH's are selected that are functional under the stringent conditions used during arraying improves the signal to noice ratio significantly.

A second element is that with VHHs it is relative simple to construct subtractive libraries for the arrays. The use of these subtractive libraries in general improves the resolution/sensitivity with about a factor 10.

A third element is that the proteins to be quantified are labelled only after removal of non-informative proteins. This approach improves the resolution/sensitivity with a factor of about 10.

The excellent quality and diversity of a naive library of single chain domain antibodies of *Camelidae* which is the preferred source of making the protein arrays according to the present invention, is shown in Example 9 below. In contrast to the state of the art, the naive library of single chain domain antibodies of *Camelidae* recognized more than 95% of a wide range of antigens. Only because of its extreme diversity the present inventors were able to develop a protein array that recognized nearly all human proteins and their post-translational modifications.

An additional advantage of carrying out the array at elevated temperature (>45°C) and/or high salt (> 1 mol) and/or in the presence of surfactants is that protein protein interactions that often occur in cells and extracellular matrices are broken down so that indeed individual proteins are measured and not, as is often the case in other protein arrays undertemined complexes of proteins.

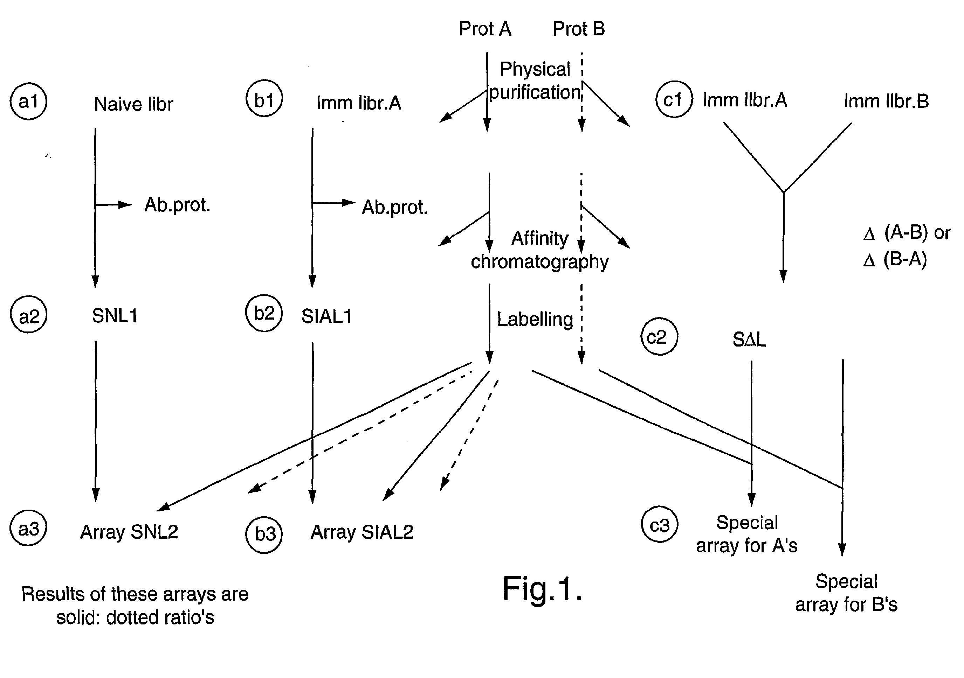

Several ways are provided to eliminate the non-informative abundant proteins from the extracts and to obtain VHH's that are relevant to the cell-, serum- or tissue extract to be investigated. The various approaches on how to select subtractive libraries of antibodies and to label only the interesting low abundant proteins for detection are summarized in Figure 1.

In a preferred method a column is used with covalently linked VHH's that recognize highly abundant proteins. These abundant proteins are bound on this affinity column. Typical examples of abundant proteins which have been removed in this way are given in Table 1 below. The remaining antigens are then preferably bound to VHH's from a naive VHH library having particular properties, as herein described. From this naive library the VHH's that recognize abundant proteins are removed, thereby creating a library as indicated in Fig 1/a1 ("sub N. libr.").

**Table 1**

| Abundant proteins, various isoforms of: | |
|---|---|
| Collagens | Tubilin |
| Fibronectin | Spectrin |
| Laminin | Int. filament |
| Elastin | Histones |
| Actin | Ribosomal proteins |
| Myosin | |

In a subsequent step (Fig 1/a2) the VHH's of the same naive library are selected for binding to low abundant proteins under conditions comparable with those used during the protein-array assay. This means conditions under which aspecific binding of antigens to support material and binding of antigens non-specifically to antibodies (or "non-cognate") are minimal. The latter two steps are achieved by specific blocking of support material and binding conditions that are normally considered as stringent conditions, such as binding in the presence of high salt and/or high surfactant concentration and relatively high temperature. The VHH's that are used for the protein array are members of the library designated as in Fig 1.

One of the approaches to detect differences between cell-type A and cell-type B, or tissue-type C and tissue-type D (not illustrated in Figure 1), can, for example, be optimized as follows (see step a3 in Figure 1):
1°.Removal of abundant proteins of both type A and B cells (or type C and D tissues, respectively) from a protein extract, first by physical and/or chemical treatment, e.g. centrifugation followed by column chromatography using a column of immobilised *Camelidae* antibodies against these abundant proteins, and/or using an elevated temperature in the range of about 20-90°C, preferably of 30-70°C, most preferably of 45-65°C, where the protein extract preferably contains a salt concentration of about 1-4 mol, for example of NaCl, most prefarably 1,3-3 mol NaCl, and optionally an anionic or nonionic; and
2°. Labelling the remaining proteins of cell-type A (or tissue-type C) with a first marker P and of cell-type B (or tissue-type D) with a second marker Q, respectively, and determining the ratio of P:Q on the array using the VHH's recognizing the low abundant proteins.

It will be evident that the VHH's selected for binding abundant proteins can be used for the development of an array. Such an array is used to determine the quantity of these proteins directly after the total extract is labelled. The fact that low abundant proteins are present does not pose a problem, neither for the labelling nor for arraying and detection.

Another approach is to start with an immune library against a certain cell or tissue extract. From these extracts the abundant non-informative proteins are removed, thereby creating library designated as in Fig 1. A protein-array is made of VHH's (from library designated as in Figure 1) that recognize low abundant proteins of relevance of the cell or tissue extract to be evaluated. The detection of relevant proteins of this cell-, serum- or tissue extract is done in a way similar to that described in the previous paragraph on arrays originating from naive libraries. Also in this approach an array is developed that is able to detect the abundant proteins in extract A.

A third approach is raising an immune bank against extracts of cell A (or tissue C), selection of the VHH's from this immune bank that bind to the proteins present in this extract and subsequent removal of the VHH's that bind to proteins of a reference cell B (or tissue D), thereby creating a subtractive library that only contains VHH's recognizing proteins that are unique for cell A (or tissue C). See Figure 1/c1. This library is designated as SI(A-B)L.

Similarly a bank SI (B-A) L is created containing VHH's that are recognizing proteins that are unique for cell B or tissue B. From these libraries arrays can be made as described before and labelling of the proteins in extract A is done after removal of the proteins also present in B, whereas labelling of the proteins of extract B is done after removal of the proteins also present in A.

The type of antibody for use in the invention is a heavy-chain variable domain derived from an immunoglobulin that is naturally devoid of light chains (VHH domain), such as those that may be obtained from camelids, wherein the antigen-binding site is contained exclusively within the heavy chain variable domain. The advantages of using this type of antibody domain are, first, that the lack of a light chain variable domain means that the extra dimension of variability in the antibody repertoire resulting from the possibility of pairing different combinations of variable domains is not present, so that during the cloning step in preparing an antibody library the generation of large numbers of irrelevant antibodies by mismatching of variable light and heavy chain domains is avoided, and consequently it is possible, while keeping the library down to a manageable size, to obtain a complete naive or immune library (that is, wherein there is at least one antibody recognising any protein) and to remove irrelevant antibodies from it. A second advantage is that VHH domains have been shown to be significantly more stable than traditional antibodies, so that array devices based upon them are expected to be significantly more robust (Van der Linden et al, BBA 1431, 37-46 (1999)).

According to a further aspect, methods are provided for generating a library of antibodies with specificity for any relevant protein in a complex mixture from a naive library or a synthetic library (in which the regions of a cloned nucleic acid sequence encoding an antibody that correspond to the complementarity determining loops are replaced with random, or partially random sequences). Libraries of these types provide resources in which antibodies capable of binding to a particular antigen may occur purely by chance, and from which these antibodies may be selected. Proteins vary widely in both abundance and immunogenicity and antibodies against some of them, notably "self-antigens", for example, are likely not to be represented in an immune library. Another advantage of the naive library used for the development of the array is that because of its size and enormous diversity contain antibodies that recognize posttranslationally modified proteins, even if these modifications are small. It is well known that raising antibodies against posttranslationally modified proteins is problematic as these antigens are often unstable in the serum of animals and therefore broken down before the immunesystem start to develop the cognative antibodies. Using various sources of abundant cellular and extracellular matrix proteins to select phages or lower eukaryotes that carry on their surface VHH's, it is relative easy to remove from the naive library the antibodies that recognize abundant proteins, thereby creating a naive (substractive) library for the low abundant proteins.

An alternative method for generating a library of antibodies with specificity for any protein in a complex mixture is to select the antibodies from an immune library.

In order to obtain these antibodies with reasonably high affinity for their cognate protein antigens, it is desirable to clone sequences encoding them from an animal which has previously been immunised against a preparation comprising all of the proteins present in the chosen reference cell type, or compartment thereof. This allows the natural *in vivo* "maturation" process leading to proliferation of cells producing such high affinity antibodies against this complex protein immunogen. A library of antibody sequences cloned from such an immunised source thus contains a substantial proportion of sequences encoding antibodies with a high affinity for the proteins of interest. This approach differs radically from the traditional approach to raising anti-protein antibodies, whereby an animal is immunised with a single purified or nearly-purified protein.

Having elicited an immune response against the complete set of proteins from the reference cell type, or cell compartment, the complete set of genes encoding the heavy chain antibodies or, more preferably, the gene fragments encoding the variable domains thereof, is cloned. Methodology appropriate to this step is well known. Suitably, a cDNA library, comprising a repertoire of nucleic acid sequences each encoding a heavy chain variable domain, is generated by cloning cDNA derived from mRNA from lymphoid cells of the immunised animal in a suitable expression vector. Also the nucleic acid sequences may be derived from genomic DNA, suitably derived from rearranged B cells.

In order to facilitate the selection steps described below, the vector in which the cDNA library is cloned directs expression of the antibody genes, in a suitable host, as fusions with a protein that is targeted for localisation at the surface of the host cell or, in the case of a viral vector, at the surface of the virus particle, such that the antibody binding domain will be exposed and able to bind antigen. Accordingly, the host cell or virus particle is referred to herein as the antibody-displaying vehicle. Suitable expression systems are well known to those skilled in the art.

Preferably the vector is provided with a sequence encoding a peptide extension, for example GST and/or a His- or Myc-tag, which will be appended to the expressed antibodies at their Nor C-termini. Said peptide extension may, for example, enable the antibody, expressed on the vehicle surface, to bind to another antibody that specifically recognises the peptide extension, and this may, in turn, facilitate isolation (if the second antibody is immobilised) or detection (if the second is provided with a suitable tag) of antibody-displaying vehicles. A peptide extension may also be used to mediate immobilisation of the antibody on the support material of an array. Introduction of GST or another protein provide us with a methodology to measure and standardise the amount of antibody present at each spot in the array.

In a preferred embodiment, the antibody-displaying vehicle is a filamentous bacteriophage, particularly M13 or a derivative thereof. The technique of "phage display" using a vehicle of this type is well documented. In another preferred embodiment the antibody-displaying vehicle is a lower eukaryotic cell, more particularly a yeast cell. Again, "yeast display" is now a well-established method.

A number of approaches are available to select from a library as described above only those antibodies that are capable of binding to at least one protein in the reference cell type, or compartment thereof. The applicability of a given method depends on whether the antibody displaying vehicle to be employed is a bacteriophage or a eukaryotic cell, and on whether the reference set of proteins are soluble in aqueous solution (e.g.cytosolic, or soluble proteins from one or more types of cellular organelle such as, for example, nucleus, mitochondria, endoplasmic reticulum, peroxisomes, vacuole) or are associated with a membrane, for example the plasma membrane, of the chosen cell type.

According to one embodiment, where the antibody displaying vehicle is a bacteriophage and the reference set of proteins are soluble proteins, a preparation of said proteins is immobilised on a solid surface, so that subsequently vehicles displaying antibodies capable of binding to them can be captured at said surface, thereby allowing them to be separated from vehicles displaying irrelevant antibodies, which can be washed away. Alternatively, complexes of specific antibody-displaying vehicles with their cognate protein antigens may be formed in solution and captured subsequently at a surface. For the purposes of either of these approaches, capture at the surface is conveniently achieved by means of biotinylating the proteins in the preparation, so that they can then be bound at a surface derivatised with streptavidin. Bacteriophage selected in this way can then be used to infect a suitable *E. coli* host strain, for phage rescue. Although designed primarily with bacteriophage in mind, these methods could also be applied where the antibodies are displayed on a eukaryotic cell surface.

An alternative selection method, applicable where the reference set of proteins are soluble proteins, and where the vehicle for displaying antibodies is a eukaryotic cell, such as a yeast cell, entails placing said antibody-displaying cells in contact with a solution containing the chosen reference mixture of proteins, said proteins having previously been labelled with a detectable tag. Those cells displaying an antibody that recognises at least one protein in the reference set are consequently identifiable by means of the tagged proteins adhering to them, enabling them to be isolated by means of a suitable cell sorter. Cells selected in this way can then be amplified by culturing. Conveniently, the tag is a fluorescent group and the cells are isolated by means of a fluorescence-activated cell sorter.

A different selection strategy that may be adopted in particular where the reference proteins are associated with one of the many membrane systems in and on the cell entails capture on said surface of vehicles displaying antibodies with binding specificity for at least one protein localised at said cell surface. The cells are then isolated, bringing their captured antibody-displaying vehicles with them, while leaving irrelevant vehicles behind. Where the antibody-displaying vehicle is a bacteriophage (and therefore too small to be spun down by low speed centrifugation) the cells are usually conveniently isolated simply by means of centrifugation. The phages spun down with them can then be used to infect a suitable *E. coli* host strain, for phage rescue.

Where the vehicle for displaying antibodies is a eukaryotic cell, an alternative selection strategy may be employed for the case where the reference cell compartment comprises the membrane of the chosen reference cell type, wherein the antibody-displaying cells are labelled with a detectable tag, then placed in contact with the reference, antigen protein-bearing cells. Reference, antigen-bearing cells to which antibody-displaying cells adhere can then be selected, on the basis of the presence of the tag and the increased size of the aggregate compared to a free antibody-displaying cell, using a suitable cell sorter. Alternatively double labelled cells could be applied. Suitably the tag will be a fluorescent group that may be attached to a molecule at the surface of the antibody-displaying cell, or incorporated internally into the cell. This would enable fluorescence activated cell sorting, in combination with size selection, to be used to isolate antibody-displaying cells that are bound to reference antigen-bearing cells. Antibody-displaying cells so selected can then be amplified by culturing.

In a variant of the method, antibodies are suitably selected on the basis of their ability to any of a set of proteins obtained by a process involving cloning DNA sequences encoding structural genes from a chosen organism, extending these sequences with a nucleotide sequence encoding glutathione-S-transferase (GST) or a His6-tag, expressing the extended genes in a suitable host or by production in cell-free translation systems, and purifying the resulting fusion-proteins. The latter purification step is greatly facilitated by provision of the GST or the His6 extension. Although the function of the proteins encoded by the genes cloned and expressed in this way are not always known, they are characterized by a unique amino acid sequence. These proteins with a GST or His6 extension are optimally suited to select specific antibodies from a phage library with methods similar to those developed for high-throughput protein screening. Other proteins or peptides can be used in place of GST or His6, provided that they provide the possibility of relatively facile affinity-purification.

Also disclosed is a high-throughput selection method with those purified tagged gene products, called micropanning, which allows the simultaneous processing of large numbers of target antigens in a controlled way bio-measuring "on-line" the success or failure of selection with a phage ELISA. The selection in microtiter plate format allows the complete automation of the technology based on the computer made decisions using the read-out of the ELISA.

Compared to currently used selection methods based on biopanning (i.e. with immobilized antigen) the "micro-panning" technique as herein described enables the simultaneous processing of large numbers of target antigens in a controlled way as well as the evaluation of many "application conditions" which can be tested for selections. The microtiter plate format allows more conditions to be tested without increasing the effort. The incorporated phage-ELISA generates "on-line" information about the success or failure of a certain panning condition. This feature combined with the microtiter plate format allows the complete automation of the technology, based on computer-made decisions on the values of the phage-ELISA for continuation of a limited number of selections.

Traditionally antigens are coated in immunotubes (4 ml) and incubated with a certain input of phage particles. After incubation and washing bound phages are eluted and amplified after infection of *E. coli* cells, thereby serving as input phages for a new round of panning. Only during the following panning round, the input phages can be tested in a phage-ELISA in order to determine the enrichment of antigen specific antibody-phages compared to the non-specific ones (background), which indicates whether the previous round of selection was successful. In the next panning round, again one concentration of input phages is used but now the antigen concentration used for coating is lowered and/or binding conditions are varied depending on the final application. Recovered phages from this round are produced to serve as input phages for a third round of panning, and so on.

Following this strategy the success of a selection can only be measured in the next panning round when new input phages have been produced and are tested in a phage-ELISA. However, for practical reasons and to avoid loss of time, the following panning round is often started without this check on antigen specific enrichment. The measured enrichment is usually only based on the comparison of the number of phage eluted from the antigen coated immunotubes versus the non-coated ones. With the present micropanning method a phage-ELISA performed with the input phage on antigen-coated and non-coated wells indicates immediately which selections are successful and are to be continued, while the less promising panning experiments can be skipped.

The novel micro-panning strategy as herein described is based on the combination of panning in a microtiter plate format and the predictive value of a phage-ELISA, carried out simultaneously. By this unique set-up the antigen specific enrichment after each round of panning can be measured directly, thereby allowing a well founded choice of input phages for following panning rounds, while experiments yielding non-enriched phages can be skipped.

Another important advantage is that due to the fact that many different conditions can be tested, varying amounts of input-phages can be used simultaneously in order to decrease the enrichment of sticky phage-antibodies. By dilution of input phage, high affinity phage-antibodies can compete more effectively with non-specific or low affinity phage-antibodies. Hence, the number of bound low-affinity or non-specific phage-antibodies will drop relatively faster than the number of the high-affinity antibodies when lower numbers of input-phage are used. Thus, compared to current panning methods micro-panning is not only a matter of scaling down, but the key difference in the working principle is that micro-panning is driven by lowering the number of non-specific phage-antibodies, whereas current panning methods are focussed on increasing the number of specific ones. Although the final goal is the same the traditional panning method is more susceptible to "sticky" phage-antibodies which can increase during panning and thereby totally drive out the specific ones, especially when used at high titers.

Therefore, micro-panning is an effective tool for selecting both naive, synthetic and immune libraries on large numbers of different target molecules, thereby enabling the generation of large panels of antibodies in rather short time frames needed for the generation of arrays (proteomics). The format of the method allows automation for high throughput panning without the need for sophisticated robotics.

Following antibody selection by any of the above methods, the next step in isolating individual genes encoding antibody fragments that are capable of binding to proteins in the reference cells is, preferably, to confirm binding by means of an ELISA or other immunoassay. In the case where the reference proteins are soluble, an ELISA, for example, can be performed by immobilising the antibodies on the walls of a microtitre plate and incubating them with a sample of the reference protein mixture, wherein said proteins have previously been labelled with a suitable tag. After allowing time for binding to occur, and washing away unbound proteins, an enzyme is added, bearing a second tag which specifically binds to the first one so that, provided binding is quantitative, one enzyme molecule is bound at the surface for every antigen protein molecule bound there by a cognate antibody. The amount of bound enzyme can then be determined by measuring its activity in catalysed a conveniently followed reaction. Conveniently, the reference proteins are tagged with biotin and the enzyme with streptavidin. The enzyme may, for example, be horseradish peroxidase.

In the case where the reference proteins are located on the surface of the chosen cell type, a different method for confirmation of binding is appropriate. For example, a sample of the reference antigen-bearing cells is added to a suitable vessel, such as a microtitre plate with V-shaped wells. Free antibodies are then added to the vessel and, after incubation, the cells are spun down and unbound material removed. After repeating the process to ensure thorough removal of unbound material, the presence of antibodies that have bound to the target cells is detected. This may be done in various ways, which will suggest themselves readily to those skilled in the art. For example, if the antibodies are expressed with a peptide extension ("TAG"), they can be detected by an ELISA using a biotinylated anti-TAG antibody and a streptavidin-enzyme conjugate.

It is preferable, for many applications of an antibody library constructed as described herein, to identify the specific proteins to which individual selected antibodies are capable of binding or, where said proteins are not already known, to characterise them as far as possible. This may be approached in a number of ways. For example, standard techniques such as two-dimensional Western blotting, followed by N-terminal protein sequencing, or two-hybrid screening may be applied. An alternative strategy, in the case where the antibody-displaying vehicle is a eukaryotic cell, is to culture said cells and then to incubate them with an excess of proteins from the reference cells, or compartment thereof, said proteins preferably being labelled with a detectable tag. This mixture is then spun down and washed to remove unbound proteins, before collecting the cells with the protein molecules bound to the antibodies displayed at their surface. Preferably, cells are selected for collection on the basis of bearing the detectable tag. The bound protein is then separated and purified by standard biochemical methods. It can then be characterised by known protein chemical methods; in particular, its N-terminal sequence can be determined. This can be compared with sequence databases in order to try to identify the protein, at least tentatively. It can also be used to design oligonucleotide probes or primers suitable for use in cloning the gene or cDNA encoding the protein, so that its complete sequence can be determined.

The approaches based on subtractive methods are particularly suitable where the antibody-displaying vehicles are bacteriophages and the protein antigens are soluble; in this case the selected phages can be used to infect a suitable *E. coli* host strain, for phage rescue. A similar method can also, in principle, be applied where the antigen-displaying vehicles are eukaryotic cells

In another embodiment applicable especially where the antibody-displaying vehicle is a eukaryotic cell and the protein antigens are soluble, the proteins from the first and second cell types are separately labelled with a detectable tag such as a fluorophore. The antibody-displaying vehicles of the parent library are incubated with the labelled proteins from the second cell type and vehicles to which any protein binds are identified by the presence of the tag and removed, using a suitable cell sorter. The remaining vehicles are now incubated with the labelled proteins from the first cell type, and vehicles to which any protein binds are identified by the presence of the tag and collected, while unlabelled cells are discarded, using a suitable cell sorter. Where the tag is fluorescent, a fluorescence-activated cell sorter is suitable for this purpose. The collected cells comprise a library wherein the antibodies are specific for proteins present in the first but not the second cell type; individual selected cells can then be amplified by culturing.

In a variant of this method, applicable where the antibody-displaying vehicles are eukaryotic cells and the chosen set of protein antigens in the first and second cell types are localised on the plasma membrane, the antibody-displaying vehicles are incubated with an excess of antigen-bearing cells of the second type, labelled with a detectable tag. The label, which may suitably be a fluorophore, may be attached to molecules at the surface of the cells, or it may be incorporated internally. Cell sorting is then carried out, so that any cells or cell aggregates bearing the label are discarded, vehicles not capable of binding to any protein on the surface of the second cell type being thereby selected. These collected vehicles are amplified by culturing and then incubated with antigen-bearing cells of the first chosen type, said cells being labelled with a detectable tag. Cell aggregates comprising both antibody-displaying vehicles and antigen bearing cells are then selected, on the basis of the presence thereon of the detectable tag (using a fluorescence-activated cell sorter, where said tag is fluorescent) and of their greater size than that of antigen-bearing cells lacking bound vehicles. As an alternative to this last step, of separation on the basis of size of bound from free antigen-bearing cells, it is possible to provide the antibody-displaying vehicles with a label, distinct and detectable independently from that borne by the antigen-bearing cells, and then to select only aggregates of cells carrying both types of label.

A complementary library, wherein every antibody is capable of binding to a protein that is present in the second chosen cell type, or compartment thereof, but absent from the first cell type, or compartment thereof, is generated by application of any of the above methods, differing only in that the role of the first and second cell types, or the proteins therefrom, are reversed.

Following antibody selection by any of the above methods, the next step in isolating individual genes encoding antibody fragments that are capable of binding to proteins from a first but not a second cell type is, preferably, to confirm the selectivity and affinity of binding by means of ELISA or other immunoassays. These immunoassay methods depend on the ability to produce the cloned antibody in soluble form and to immobilise it at a suitable surface. These steps, as well as the assays themselves may be carried out in the same ways as described above for the case of a complete antibody library. Antibodies meeting the selection criteria should demonstrate reasonable affinity for a protein amongst those from the first cell type, but not for any protein from the second cell type.

A subtractive antibody library is particularly valuable for providing antibodies capable of binding to, and hence permitting identification and characterisation of, proteins that are present in differing amounts in cells of different types. In particular where the library is complete, in the sense that it comprises antibodies specific for all of the proteins present in a significantly greater amount in the first cell type, or the chosen compartment thereof, and where this library is complemented by a second one wherein there are antibodies capable of binding to every protein that is present to a significantly greater extent in the second cell type, or compartment thereof. This provides a very powerful means of identifying the key metabolic differences that underlie different properties of the two cell types. This approach is applicable to a wide variety of pairs of cell types, of which a few include cells from different but related species, cells from alternatively differentiated cells from within an organism, nominally equivalent cells from organisms showing genetic or developmental differences, or normal cells in comparison with others affected by disease, ageing or drugs.

Isolation of the specific proteins to which individual selected antibodies bind, in order to characterise and, where possible, identify them may be approached in a number of ways, such as those described above for the case of the complete antibody library. Once a set of proteins that are present in significantly amounts in the alternative cell types has been identified, one possibility is to use the presence or to exploit the activity of one or more of these for diagnostic purposes in, for example identifying the presence of disease. It may, indeed, be convenient to use the antibody, from the subtractive antibody library, to which said protein binds as the basis for an immunoassay.

It is also possible to investigate the possible role of individual proteins, identified through their binding to antibodies in a suitable subtractive library, in bringing about the metabolic differences observed between two cell types. Where it is desired to reverse or ameliorate said differences, for instance where one of the cell types is in a disease state, this provides a way to identify possible target molecules, for example for drug therapy. This may be approached, for example, by suppression of expression of the gene encoding the protein, in cells where it would otherwise be present, or transformation of cells in which it would otherwise be absent with a construct containing the gene and a promoter to direct its expression. Alternatively, a convenient way to suppress the activity of the protein in question is to generate a transgenic cell in which the gene encoding an antibody from the subtractive library, said antibody being capable of binding to the protein in question, is expressed and the antibody directed to the cell compartment where the protein is ordinarily active. The observed phenotypic changes can provide powerful insights into the relevance of the protein for a condition in which its abundance has been observed, by means of the generation of the subtractive antibody library, to be diminished.

In a further aspect, samples of multiple individual antibodies from either a complete or a subtractive antibody library according to the invention are immobilised at distinct positions in an array on a solid surface. This array may then be exposed to a preparation containing the proteins from a chosen cell type or cell compartment which it is desired to characterise, so that for those antibodies whose cognate protein antigens are present, binding occurs at the solid surface. Binding can be assessed most conveniently by tagging the proteins in the preparation to be characterised with a readily detectable label, such as a fluorescent or other optically detectable chemical group, or a metal (in particular gold or silver) or a radiolabel, so that the presence of bound material is revealed by the accumulation of the tag at the loci of individual antibodies in the array. The pattern of binding may be assessed particularly effectively where the antibody array is immobilised on a chip suitable for reading with an optical imaging device.

Immobilisation of the antibodies on the surface may be achieved through covalent coupling or through non-covalent interactions. To this end, the antibodies may be derivatised with any suitable chemical groups, provided that this does not interfere with their binding capabilities, and they may optionally be provided with a peptide extension, encoded at the DNA level, through which coupling may conveniently be achieved. In a preferred embodiment, the antibodies are biotinylated, thus allowing them to be bound at a surface derivatised with streptavidin. Biotinylation may be carried out *in vitro,* by conventional methods, or *in vivo*, by providing the antibodies with a suitable peptide extension, where the sequence of said extension has been found to specify biotinylation in the host species in which the antibodies are expressed (Schatz, Biotechnology 11, 1138-1143 (1993)).

Antibody arrays can be constructed with any set of antibodies. The identity, or at least the sequence, of the protein to which each individual antibody is able to bind is preferably known. These arrays make it possible to ascertain which of these protein antigens are present, and approximately in what amount, in a sample of unknown protein content. For example, once a complete antibody library for one cell type, or compartment thereof, has been constructed and incorporated into an array, it is then possible to compare the distribution of proteins in a second, phenotypically different cell type: if, for example, a protein that was present in the reference cell type is also present in the second cell type, this can be revealed by detecting binding to a cognate antibody in the array.

It is further possible to gain an approximate indication of the relative abundance of individual proteins in two related samples, such as two different cell types or cells with different histories. For example, the proteins in one said sample may be labelled with a detectable tag while the proteins in the second said sample are labelled with a different, distinguishable tag. A mixture of the two samples is then placed in contact with an antibody array according to the invention and the relative amounts of the alternatively tagged proteins bound to individual antibodies in the array are determined. For example, if the tags are fluorescent groups, this may be achieved by measuring the intensity at the respective emission maxima of the alternative tags. Differences in protein expression between cell types, for example between cells of different age, can be probed by these methods and the results compared with the those obtained using gene arrays.

The concept embodied in this disclosure of using an array of antibodies to provide a profile of a chosen cell type or compartment thereof, in terms of the proteins that are present in said cell or compartment clearly bears comparison with the use of DNA arrays, which are now becoming established as a tool for characterising the distribution of mRNA species in a chosen cell type. Similar array imaging technology may be applied in both cases, although the underlying biological principles of the methods and the techniques for constructing the arrays are entirely different. In both cases, the aim is to obtain a perspective on the full range of metabolic activities in the chosen cell type, as reflected in the proteins that are present to control and facilitate them. The key advantage of the antibody array is that it allows a direct, and semi-quantitative assessment of the protein content of a given cell type and it is this which determines the functional properties of the cell, much more directly than the amounts of different mRNAs that may be present. Further, since proteins are generally much more stable than mRNAs, the results obtainable with the antibody array would be expected to be less dependent on the details of the experimental protocol followed. Still another advantage is that the robustness of the antibodies means that arrays based on these can be used several times, with complete removal of bound antigens in between, without loss of quality of the results obtainable. Using VHH in the array provides a number of advantages, such as an improvement of sensitivity/resolution in the order of 10 to 100 times, and detection of post-translationally modified proteins.

The following examples are provided by way of illustration only.

### EXAMPLE 1. Preparation of cytoplasmic proteins from S. cerevisiae cultivated under different conditions

One litre of YNB medium (0.67 % Yeast Nitrogen Base) containing 2% glucose and another litre of YNB medium with 2% galactose were inoculated with *Saccharomyces cerevisiae* and grown until an OD660 of 1 was reached. Cells were harvested (10 minutes 7,000 x g) and resuspended in 40 ml phosphate buffered saline (PBS). Cells were lysed at 20,000 Psi in a French press. Whole cells and cell walls were removed from the lysates by centrifugation. Membrane fractions and ribosomes were removed from the supernatants by ultra-centrifugation at 100,000 x g for 60 minutes. The clear lysates contain all soluble intracellular proteins at a total protein concentration of approximately 10 mg/ml.

### EXAMPLE 2. Induction of a humoral immune response in Ilama.

A female Ilama was immunised with galactose grown yeast extract (YEgal) in phosphate buffered saline (PBS) subcutaneously and intramuscularly. Per immunisation 2 ml was injected containing respectively 5 mg, 5 mg, 2.5 mg and 1.25 mg YEgal. Immunisations were performed according to the following time schedule: the second immunisation was performed four weeks after the first injection and the third immunisation again four weeks after the second one. The immune response was followed by titration of serum samples in ELISA with YEgal immobilised on Nunc maxi-sorb plates (coat solution 10 µg/ml YE diluted in PBS). After incubation with serum, the bound Ilama antibodies were detected with polyclonal rabbit-anti-llama antiserum (obtained via immunising rabbits with Ilama immunoglobulins purified via ProtA and ProtG columns; ID-DLO) and swine-anti-rabbit immunoglobulins (DAKO) conjugated to horseradish peroxidase. Finally the peroxidase enzyme-activity was determined with tetramethyl benzidine and urea peroxide as substrate and, after termination of the reaction by adding H₂SO₄, the optical density was measured at 450 nm.

Western blots containing the proteins from YEgal and YEglu were incubated with pre- and postserum antibodies and revealed a strong response against the whole spectrum of proteins after immunisation.

### EXAMPLE 3. Cloning, selection and screening of Ilama VHH fragments

### 3.1 Isolation of (VHH fragments against YEgal proteins.

From the Ilama, positively responding against YEgal as tested in ELISA, a blood sample of about 200 ml was taken and an enriched lymphocyte population was obtained via centrifugation on a Ficoll (Pharmacia) discontinuous gradient. From these cells, total RNA was isolated by guanidinium thiocyanate extraction (e.g. via the method described by Chomczynnski and Sacchi (1987), Analytical Biochem., 162, 156-159). After first strand cDNA synthesis using MMLV-RT (Gibco-BRL) and random oligonucleotide primers (Pharmacia), DNA fragments encoding VHH fragments and part of the long or short hinge region were amplified by PCR using specific primers:

The DNA-fragments generated by PCR were digested with *Pst*I (coinciding with codon 4 and 5 of the V_{H}H domain, encoding the amino acids L-Q) and *Not*I (introduced at the 5' end of the hinge specific oligonucleotide primers, coinciding with the amino acid sequenceA-A-A), and cloned in the phagemid vector pUR5071 as gene-fragments encoding the V_{H}H-domain including the hinge region fused to the geneIII protein of the *E*. *coli* bacteriophage M13, thereby enabling display of the antibody fragment on the surface of the filamentous phage (McCafferty et al (1990), Nature, 6, 552-554).

### 3.2 Enrichment of Orotidine-5-monophosphate-decarboxylase (OMCase) binding V_{H}H domains via phage display methodology

A display library with 1x10⁹ clones, of which 75% contained a complete V_{H}H encoding insert, was constructed in phagemid vector pUR5071. Phage particles exposing V_{H}H fragments were prepared by infection of *E*. *coli* cells harbouring the phagemid with helper phage VCS-M13 (Marks et al (1991), J. Mol. Biol., 222, 581-597). By precipitation of phage from the culture supernatant with PEG6000, free V_{H}H fragments were removed, thereby avoiding a disturbing competition for binding to antigen between phage bound and free V_{H}H domains. Phage antibodies were incubated with in vitro biotinylated OMCase. Antigen-antibody complexes arid associated phage particles were pulled out of the solution with streptavidin coated magnetic beads (DAKO) (see Hawkins et al (1992), J. Mol. Biol., 226, 889-896). After an extensive washing procedure, *E*. *coli* phage was eluted from the beads with 0.1 M triethylamine (Baker) by disruption of the antigen-antibody binding with this alkaline shock. After neutralisation with 0.5 volume of 1 M Tris-HCl pH7.4, phage was rescued by transfection into the *E. coli* host TG1. A renewed selection was performed with phage prepared from the transfected population of *E. coli* bacteria as was described before.

Individual *E. coli* clones obtained after three rounds of selection were grown in wells of microtiter plates, and the production of was induced by the addition of isopropyl-β-D-thiogalactopyranoside (IPTG, 0.1 mM). After 16 hours of growth, the culture supernatant of the clones was analysed in ELISA for the presence of V_{H}H fragments, which specifically bind to biotinylated OMPcase immobilised on streptavidin coated ELISA plates. Bound V_{H}H fragments were detected with mouse monoclonal anti-myc antibody followed by incubation with polyclonal rabbit-anti-mouse conjugated to horseradish peroxidase (DAKO).

On western blots with purified OMCase and both extracts the enzyme was recognised by the obtained V_{H}H fragments. This example shows that antibody fragments can be selected from the library even against proteins, which are present in low concentrations.

### 3.3 Enrichment of YEgal protein binding V_{H}H domains via phage display methodology.

The phage display library described in section 3.2 was also used for the isolation of antibody fragments recognising YEgal proteins. Phage particles displaying the antibody were purified by PEG-precipitation and subsequently incubated with in vitro biotinylated YEgal proteins. Antigen-antibody complexes and associated phage particles were pulled out of the solution with Ultralink™ immobilized streptavidin Plus (Pierce). Ultralink was used instead of dynabeads because of the higher binding capacity. After an extensive washing procedure, *E. coli* phage was eluted from the column material with 0.1 M triethylamine. After neutralisation phage was rescued by transfection into the *E. coli* host TG1. A renewed selection was performed with phage prepared from the transfected population of *E. coli* bacteria as was described before.

Individual *E. coli* clones obtained after the two rounds of selection were screened for production of V_{H}H fragments recognising antigens present in the YEgal protein extract. The V_{H}H fragments present in the culture supernatant were captured by monoclonal anti-MYC antibody coated on ELISA plate. After incubation with biotinylated YEgal extract specifically bound biotinylated proteins were detected with streptavidin-conjugated horseradish peroxidase (BIORAD).

### 3.4 Enrichment of galactose specific protein binding V_{H}H domains via counter selection with YEglu and phage display methodology.

Phage particles were produced as described above in section 3.11. Phages were incubated with an excess of (non-biotinylated) YEglu before biotinylated YEgal was added. Antigen-antibody complexes and associated phage particles were pulled out of the solution with Ultralink™ immobilized streptavidin Plus. After an extensive washing procedure to remove all non-bound phage particles and all phage particles associated with non-biotinylated proteins, *E. coli* phage was eluted from the column material with 0.1 M triethylamine. After neutralisation phage was rescued by transfection into the *E. coli* host TG1. A renewed counter-selection was performed with phage prepared from the transfected population of *E. coli* bacteria as was described before.

Individual *E. coli* clones obtained after two rounds of counter-selection were grown in wells of microtiter plates, and the production of V_{H}H fragments was induced by the addition of isopropyl-β-D-thiogalactopyranoside. After 16 hours of growth, the V_{H}H fragments present in the culture supernatant of the clones were captures via immobilized monoclonal anti-MYC antibody. An incubation with biotinylated YEgal or in the duplicate well with biotinylated YEglu followed. Bound biotinylated proteins were detected with streptavidin conjugated horseradish peroxidase (BIORAD).

### EXAMPLE 4. Selection of VHH fragments against all ORF's of S. cerevisiae using robotics

### 4.1 Strategy for selection and screening.

From the collection of yeast clones, expressing all 6200 ORF's as GST fusion proteins, the antigens for selections were purified with Gluthathione-Uniflow Resin (Clontech). The purification was performed on pooled cultures, thereby yielding a mixture of different ORF-GST-fusion products. For this, an equal volume of each culture from the 96 well masterplates containing the DMSO stocks (Genetic Research) was taken as inoculum for starting of one culture. The purified mixture was coated for biopanning, while free GST was added to the phage solution, thereby loosing the clones producing GST specific VHH fragments. After a single selection round a large number of clones were picked with a robot and arrayed on filters for analysis of their specificity as was described (De Wildt et al., Nature Biotechnology, 18 (2000)). After growth of the colonies on plates containing glucose to repress antibody production, the filters were removed and transferred to new agar plates for induction with IPTG. Between the sheet containing the colonies and the surface of the plate a number of other nitrocellulose filters containing different antigens and blocked with an irrelevant protein, such as BSA, was placed; the antigen containing filters can capture antigen specific antibody fragments, which diffuse through these set of filters. The bound VHH fragments can be visualized by means of their tag-sequences or with a rabbit-anti-VHH polyclonal antibody, thereby revealing which antibody produced by the arrayed clones recognizes a certain ORF. Preferably filters are incubated with twenty pools of ORF's, made up of the collected clones from the eight columns and twelve rows of the 96 wells master plate. By using twenty filters large numbers of clones can be screened against 96 antigens, yielding sufficient information for deducing the antigen recognition of all individual VHH fragments. By using these methods the large numbers of different VHH fragments, which are needed to generate the antibody arrays, are rapidly selected and screened.

### 4.2 High-throughput selection methodology

This section exemplifies the so-called micro-panning selection method described above in the Detailed Description. Microtiter plate wells were coated with antigen solution (starting at 100 µg/ml per well for first round of selection with the Ilama/camel naive library) and after blocking incubated with input-phage, which can be added in serial dilutions (10-fold dilutions, 100 µl/well in 2% marvel, 1%BSA, 0.05% Tween-20 in PBS, pH 7.4). As a negative control the same samples of input-phage were added to non-coated wells. The microtiter plates were incubated for one to two hours on a microplate shaker and subsequently washed (15x PBS-T, 3x PBS, 250 µl/well). After washing half of the plate was eluted with 100 µl 0.1 M triethylamine for 20 minutes. Eluted samples were neutralized with 50 µl 1 M Tris-HCl pH 7.5. Eluted phage was recovered by adding 75 to 150 µl eluate to 600 µl 2TY medium and 250 µl TG-1 cells followed by incubation at 37°C for 30 minutes. After incubation 100 µl of each sample was plated out on LB amp/glu agar plates. The remaining cells were centrifuged and pellets re-suspended in 5 ml 2TY amp/glu, and grown for 16 hours in a shaker at 37°C. From these cultures, glycerol stocks were made or phage was produced for a next selection round.

After elution of the phage from half of the microtiter plate, the whole plate was washed with PBST and incubated with rabbit-anti-p8 serum (1:5,000) for one hour at RT. The bound anti-p8 antibodies were detected with swine-anti-rabbit IgG HRP conjugate (DAKO).

The results from the selections with a self-antigen, VHH-fragment 2E3 recognizing a protein in a tomato extract, using a Ilama single-domain library, are shown in Table 2. In the first round several dilutions (1 to 1000-fold) of input phage were used, which illustrate that a lower input can give lower backgrounds (compare 1x with 10x), while the number of eluted phage from the 2E3 coated tube did not change. The number of eluted phage correlates well with the read-out of the phage-ELISA. For the second round of panning the output phage from the different dilutions of the first round were used, again in several dilutions. The best results, i.e. highest output vs. lowest background, were found when the 10-fold dilution from the first round was used as input.

**Table 2 Results from the selection on a naive Ilama library with VHH 2E3**

| (A) Panning Round 1 | | | | |
|---|---|---|---|---|
| (VHH 2E3 coated at 50 µg/ml) | | | | |
| **No. Input Phages** | **Phage ELISA (OD450)** | | **No. of Eluted phages** | |
| | 2E3-VHH | No Coat | 2E3-VHH | No Coat |
| R1 (1x) = 9 x 10e11 | 0.204 | -0.017 | 8 x 10e3 | 660 |
| R1 (10x) = 9 x 10e10 | 0.039 | -0.001 | 8 x 10e3 | 70 |
| R1 (100x) = 9 x 10e9 | 0.007 | -0.001 | 500 | 30 |
| R1 (1000x) = 9 x 10e8 | -0.009 | -0.015 | 220 | 0 |

| (B) Panning Round 2 | | | | |
|---|---|---|---|---|
| (VHH 2E3 coated at 10 µg/ml) | | | | |
| **No. Input Phages** | **Phage ELISA (OD450)** | | **No. of Eluted Phages** | |
| R1 (1x) | 2E3-VHH | No Coat | 2E3-VHH | No Coat |
| R2 (10x) = 1 x 10e11 | 1.870 | 0.002 | ± 10e6 | 500 |
| R2 (100x) = 1 x 10e10. | 2.003 | -0.015 | ± 10e6 | 10 |
| R2 (1000x) = 1 x 10e9 | 2.116 | -0.019 | ± 10e5 | 10 |
| R2 (10000x) = 1 x 10e8 | 1.200 | -0.016 | ± 10e4 | 0 |

| **No. Input Phages** | **Phage ELISA (OD450)** | | **No. of Eluted Phages** | |
|---|---|---|---|---|
| R1 (10x) | 2E3-VHH | No Coat | 2E3-VHH | No Coat |
| R2 (10x) = 8 x 10e10 | 1.602 | 0.002 | ± 10e6 | 360 |
| R2 (100x) = 8 x 10e9 | 2.558 | -0.015 | ± 10e6 | 60 |
| R2 (1000x) = 8 x 10e8 | 2.119 | -0.015 ± | 5 x 10e5 | 10 |
| R2 (10000x) = 8 x 10e7 | 1.340 | -0.013 ± | 5 x 10e4 | 0 |

| R1 (100x) | 2E3-VHH | No. Coat | 2E3-VHH | No Coat |
|---|---|---|---|---|
| R2 (10x) = 4 x 10e10 | 2.112 | 0.000 | ± 10e6 | 600 |
| R2 (100x) = 4 x 10e9 | 2.605 | -0.015 | ± 10e6 | 150 |
| R2 (1000x) = 4 x 10e8 | 2.040 | -0.017 | ± 10e6 | 0 |
| R2 (10000x) = 4 x 10e7 | 0.758 | -0.016 ± | 5 x 10e4 | 10 |

The second example shows the results from the selection with the azo-dye (RedReactive-6; RR6) coupled to BSA. Rather low OD's were found in the first round of selection with the phage-ELISA corresponding with low numbers of eluted phage with the best proportion of RR6-specific phage vs. background in the 10-fold diluted input (Table 3). The second round of panning gave much higher signals in the phage-ELISA as well as higher numbers of eluted phage with approx. 10 % background. A rather high fraction of the fragments obtained after round 2, which were analysed in ELISA, turned out to react with the dye (>50%), while a low number of antibodies recognizes the carrier-protein BSA (10%).

**Table 3**

| Results from the selection on a naive Ilama library | | | | |
|---|---|---|---|---|
| with RR6-BSA | | | | |
| (A) Panning Round 1 | | | | |
| (RR6-BSA coated at 50 µg/ml). | | | | |
| **No. Input Phages** | **Phage ELISA (OD450)** | | **No. of Eluted Phages** | |
| | BSA-RR-6 | No Coating | BSA-RR-6 | No Coating |
| R1 (1x) = 9 x 10e11 | 0.026 | -0.017 | 770 | 660 |
| R1 (10x) = 9 x 10e10 | 0.013 | -0.001 | 400 | 70 |
| R1 (100x) = 9 x 10e9 | 0.007 | -0.002 | 0 | 30 |
| R1 (1000x) = 9 x 10e8 | 0.006 | -0.015 | 10 | 0 |

| (B) Panning Round 2 | | | | |
|---|---|---|---|---|
| (RR6-BSA coated at 10 µg/ml) | | | | |
| **No. Input Phages** | **Phage ELISA (OD450)** | | **No. of Eluted Phages** | |
| R1 (1x) | BSA-RR-6 | No Coating | BSA-RR-6 | No Coating |
| R2 (10x)= 1 x 10e11 | 1.925 | 0.955 | ± 10e6 | ± 10e5 |
| R2 (100x) = 1 x 10e10 | 1.876 | 0.147 | ± 10e5 | ± 8 x 10e3 |
| R2 (1000x) = 1 x 10e9 | 0.690 | 0.004 | ± 5 x 10e4 | 1250 |
| R2 (10000x) = 1 x 10e8 | 0.165 | 0.004 | ± 5 x 10e3 | 200 |

| **No. Input Phages** | **Phage ELISA (OD450)** | | **No. of Eluted Phages** | |
|---|---|---|---|---|
| R1 (10x) | BSA-RR-6 | No Coating | 2E3-VHH | No Coating |
| R2 (10x) = 7.5 x 10e10 | 1.752 | 1.148 | ± 10e6 | ± 10e5 |
| R2 (100x) = 7.5 x 10e9 | 1.738 | 0.425 | ± 10e6 | ± 5 x 10e4 |
| R2 (1000x) = 7.5 x 10e8 | 0.474 | 0.042 | ± 5 x 10e4 | ± 5 x 10e3 |
| R2 (10000x)=7.5 x 10e7 | 0.129 | -0.006 | ± 5 x 10e3 | 1500 |
| | | | | |
| (C) ELISA screening with soluble VHH on binding to RR6-BSA conjugate and BSA (coated at 10 µg/ml). | | | | |
| | | | | |

| **Panning Round** | **ELISA** **no. pos (OD450>0.4) / no. tested** | | | |
|---|---|---|---|---|
| | RR-6 | BSA | | |
| R1 (1x) | 0 / 16 | 0 / 16 | | |
| R1 (10x) | 1 / 16 | 1 / 16 | | |
| R1 (1x) / R2 (10.000x) | 13 / 24 | 0 / 24 | | |
| R1 (10x) / R2 (10.000x) | 12 / 24 | 2 / 24 | | |

### EXAMPLE 5.

### 5.1. Preparation of proteins from healthy and diseased muscle tissues

A biopt of muscle tissue was homogenised with a potter in 1 ml of buffer. Membranes were pelleted by low speed centrifugation. The membrane fraction was resuspended in 1 ml of buffer. By using this protocol a cytosolic fraction was obtained containing 4.5 mg protein and a membrane fraction with 1 mg of protein. The quality of the fractions was judged on a Coomassie stained gel, revealing entirely different sets of proteins for the cytosolic and membrane fractions.

### 5.2. Induction of a humoral response in Ilama against muscular proteins

Similar protocols as described in Example 2 are used; the llama was immunised four times with a mixture of 600 µg of cytosolic protein and 125 µg of membrane protein per injection.

### 5.3. Cloning, selection and screening of Ilama VHH fragments raised against muscle tissues.

Similar protocols as described in Example 3.1 are used. Selections with in vitro biotinylated cytoplasmic proteins was performed, while biopanning with membrane proteins was achieved by dissolving the suspension in urea (8 M in PBS) and use of this stock solution diluted in PBS for coating of an immuno-tube.

### 5.4. Selection on a subset of muscular proteins expressed as epitope-tagged products

Of particular interest for the muscle dystrophy, FSHD and other muscular dystrophies, are the following proteins, of which tagged versions have been constructed according standard procedures:
FRG 1 (facioscapulohumeral muscular dystrophy Region Gene 1)
FRG 2 (FSHD Related Gen 2)
PABP 2 (Polyadenylate Binding Protein 2)
Emerin (nuclear membrane protein, Emery Dreifuss muscular distrophy);
Calpain-3 (limb-grindle muscular dystrophy type 2A)
Caveolin 3 (limb-grindle muscular dystrophy type 1C)
Desmin (Desmin storage myopathy)
SIR2L (human SIR2 homologue)

The following tagged proteins are of interest not only for muscular dystrophies;
HP1α (*Drosophila* heterochromatin protein homologue)
HP1β (*Drosophila* heterochromatin protein homologue)
HP1γ (*Drosophila* heterochromatin protein homologue)

These tagged proteins were used for selections according to the protocol described in Example 4.

### EXAMPLE 6.

### 6.1. Preparation of proteins from CaCo-2 cells (a cell type of intestinal epithelial tissue)

CaCo-2 cells were plated in 185 cm culture flasks; medium (DMEM supplemented with 20% H.I. Foetal Calf Serum and gentamycin) was changed every other day. After one month of culturing cells were harvested by washing two times with PBS and scraping in PBS-inh (with protease inhibitors). After centrifugation (5 minutes at 700 xg), the cell pellet was resuspended in15 ml PBS and lysed by passing ten times through a 22G syringe. Low speed centrifugation (5 minutes at 700 x g) yielded a supernatant fraction with the cytosolic proteins (38.9 mg). The pellet was again passed through the syringe and centrifuged at high speed (60 minutes at 10,000 x g in a SS34 rotor) to yield the membrane bound proteins in the pellet (28.2 mg) and additional cytosolic proteins (6.1 mg) in the supernatant.

### 6.2. Induction of a humoral immune response in Ilama against CaCo-2 proteins

Similar protocols were used as described in Example 3.1.

### 6.3. Cloning, selection and screening of Ilama VHH fragments raised against CaCo-2 proteins

Similar protocols were used as described in Example 3.1. Western blot analysis using the extracts boiled in reducing sample buffer showed that the antibodies of the immunized Ilama recognize large numbers of different proteins.

### EXAMPLE 7.

### 7.1. Preparation of proteins from intestinal tissue (Brush Border Membranes) of pigs

Three pigs (3 weeks post-weaning weighing 10-15 kg, coded 3602, 3611 and 3613) were bled under Nembutal anaesthesia. Brush Border Membranes fractions were prepared from intestinal segments as was described (Sellwood R. et al., J. Med. Microbiol. 8:405-411 (1975)). The small intestine was removed from the animals, and the pertinent section(s) excised, and put on ice in PBS13. The intestinal segments, each made up of a one-meter section, were taken from five different locations: (A) anterior duodenum; (B) between A and C, (C) mid jejunum, (D) between C and E, and (E) posterior ileum.

The intestinal segment was cut open, and the mucosa scraped into a beaker containing cold 0.005 M EDTA/PBS13 pH 7.4. The material was homogenized in a Waring blender during 20 sec at high speed. The resulting homogenate was sieved through medical gauze (van Heek Medical) to remove coarse particles, and subsequently centrifuged at 15 min at 700 x g at 4 °C (Sorvall, GSA rotor). The resulting pellet was resuspended in hypotonic EDTA-solution (0.005 M EDTA, adjusted to pH 7.4 with 0.5 M Na₂CO₃), and centrifuged at 800 x g, 15 min 4 °C. The washing was repeated (5-10 times) with hypotonic EDTA, while reducing the speed of centrifugation with 100 x g at each step under constant microscopic monitoring of supernatant and pellet for Brush Border Membranes (BBM). The procedure was stopped when no material was washed away from the pellet, and a microscopically pure BBM pellet was obtained.

### 7.2. Induction of a humoral immune response in proteins of intestinal tissue

For immunisation of three different Ilamas the BBM pellets from the different intestinal segments were used as follows:
- section (A) anterior duodenum, 5 ml material in total, used for immunization of Ilama 591;
- section (C) mid jejunum, 5 ml material, used for immunization of Ilama 617,
- section (D) between mid jejunum and posterior ileum (2 ml), combined with section (E) posterior ileum (2 ml), used for immunization of Ilama 2587.

The pellets (5 ml for sections (A) and (C) and 4 ml of the combined sections (D) and (E)) were diluted by adding 4 ml PBS13. From this mixture 1 ml was mixed with 1 ml PBS and 3 ml Specol. After homogenization, the total volume (5 ml) was injected in a llama, 50% intramuscularly, and 50% subcutaneously, according to the standard protocol for llama immunization. Three consecutive immunizations were performed with at month intervals. The remainder of the pellet was stored frozen at -20 °C in PBS 13/glycerol (50% (v/v)).

The immune response against the mixture of antigens was analyzed with western blot, revealing that a broad spectrum of proteins were detected by the polyclonal antibodies from the sera after immunization.

### 7.3. Cloning, selection and screening of lama VHH fragments raised against intestinal tissue

Similar protocols as described in Example 3.1 were used. Subtractive methods as described in Example 3.4 were applied to generate antibodies recognizing intestinal segment specific antigens. These antigens were identified by 2D electrophoresis and western blot combined with amino terminal sequencing and/or mass spectrometry.

### EXAMPLE 8.

### 8.1. Construction of VHH based protein arrays

Immobilisation of llama antibodies on solid surfaces, such as chips, was achieved by (non-covalent) adsorption or by directed covalent coupling by fi. using amino groups (of lysins or of the amino terminus) of the antibody fragments to activated carboxyl groups at the solid surface by conventional carbodimide coupling using 1, ethyl-3-[3-dimethyl aminopropyl] carbodiimide (EDC) and N-hydroxysuccinimide (NHS) as was described in Unilever patent (T3082). Alternatively special tag-sequences were used to direct non-covalent binding to an immobilized "receptor" molecule, which interacts with this tag. An example is the tag, which directs in vivo biotinylation of the VHH fragment by *Escherichia* coli (Schatz, PJ. Biotechnology 11, 1138-1143 (1993)), and thereby facilitates easy immobilisation without prior purification by the high affinity interaction with immobilized streptavidin.

A set of approximately 100 (VHH fragments recognizing different ORFs of *S. cerevisiae* (described in Example 4.1) was recloned in the *E. coli* production vector yielding C-MYC/His6-tagged and biotinylated fragments.

The tags used were C-MYC (bold in SEQ.ID.NO:3), recognized by monoclonal antibody 9E10 (Munro, S., and Pelham, H.R., Cell 46, 291-300 (1986)), followed by a 12-mer peptide encoding an in vivo biotinylation signal (bold and underlined) and the hexahistidin tail (italics) for purification with IMAC (Hochuli, E. et al., Biotechnology 6, 1321-1325 (1988)). The complete sequence fused to the carboxy terminus of the VHH is presented below:

### EQKLISEEDLN GAA LRSIFEAQKMEW HHHHHH

SEQ ID No:4 The VHH fragments present in bacterial supernatants were arrayed on a chip coated with streptavidin. From *S. cerevisiae* cultured under two different regimes cytosolic protein extracts (prepared as described in Example 1) were labelled with FITC and CY5 according to methods suggested by suppliers of kits (Pierce). The chip was incubated with a 1:1 mixture of both differentially labelled protein extracts and ,the response visualized after washing. A clear picture of the up- and down regulated proteins was obtained.

### 8.2 Anti-mouse Ig array

### 8.2.1 Selection of mouse IgG-subclass cross-reactive and specific VHHs

The antibodies and Fc fragments used as antigens in the selection and screening of antibody fragments are presented in Table 4 below.

**Table 4**

| Antibodies and Fc fragments used in the selection and screening experiments | | |
|---|---|---|
| Name | Subclass + LC | Source |
| α-RR6 | IgG1-κ | Unilever Research Vlaardingen |
| α-Traseolide (02/05/01) | IgG1-κ | MCA |
| α-MYC | IgG1-κ | MCA |
| α-TAG | IgG2a-κ | Unilever Research Vlaardingen |
| HOPC-1 | IgG2a-λ | Sigma |
| MOPC-141 | IgG2b-κ | Sigma |
| α-Muc | IgG3-κ | Unilever Research Colworth |
| M9019 | IgG3-λ | Sigma |
| MoFc | | Jackson Immunoresearch |
| HuFc | | Jackson Immunoresearch |

At days 0, 22, 43 and 85 llama 2590 was immunised with mouse α-Traseolide and with α-TAG mAB. Llama 2591 was immunised at days 0, 22 and 64 with constant fragments of human and mouse immunoglobulins.

After the last immunisation, the B-lymphocytes were isolated from the blood. Total RNA was extracted from the B-cells and then used as a template in random primed RT-PCR. The gene segments encoding the single-domain variable domains were amplified on random primed cDNA and cloned in pUR5071 as was described before. The size of the resulting library was approximately 10⁹.

The immunisation of llamas 2590 and 2591 resulted in an α-Mab and an α-Fc phage library, respectively. A first round of selection on the antigens mouse IgG1 and MoFc was performed with both libraries. All selections resulted in an enrichment of the phage population of 5 to 10 times.

This selection procedure was followed by two more rounds of selection. For a subsequent selection round the antigen concentration was lowered from 30 nM to 5 nM. A fraction of the output phages of round 2 was not rescued and amplified, but dialysed against PBS and directly used for a new selection round. For the third round of selection a mouse mAB was taken from an isotype different from the one used in the first two selection rounds, thereby driving the isolation of cross-reactive antibody fragments (Table 5). After each selection round, single colonies were screened in ELISA for the production of mouse IgG cross-reactive VHH fragment by (Table 5).

**Table 5**

| Selection of mouse IgG-subclass cross-reactive VHH's. | | | |
|---|---|---|---|
| In the selection α-RR6 (IgG1), α-TAG (IgG2a) and α-Muc (IgG3) were used. | | | |
| Library | Round 1 [antigen]=30 nM (enrichment) (% positive in ELISA) | Round 2 [antigen]=5 nM (% positive in ELISA) | Round 3 [antigen]=5 nM (% positive in ELISA) |
| α-Mab | 1. IgG1 (10x) (17) | 1A. (29) IgG1 | 1B. IgG2a (25) |
| | | | 1C. IgG3 (54) |
| | 2. MoFc (8x) (0) | 2A. MoFc (0) | 2B. HuFc (0) |
| | 3. Blanc | - | - |
| α-Fc | 4. IgG1(8x)(38) | 4A. IgG1 17) | 4B. IgG2a (13) |
| | | | 4C. IgG3 (0) |
| | 5. MoFc (10x)(0) | 5A. MoFc (4) | 5B. HuFc (8) |
| | 6. Blanc | - | - |

VHHs reacting positively in the ELISA screenings were tested on BIAcore for their binding to human and mouse Fc fragments, IgG1 (α-RR6) and IgG2a (α-TAG).

Two VHHs derived from the α-Mab library, C4 and G4, and two VHHs derived from the α-Fc library, C7 and E7, were further analysed. The choice for these VHHs was based on the high response shown in the BIAcore experiment and on the cross-reactivity these VHHs showed by binding to two different antibodies or Fcs. The selected VHH were produced in *E. coli* both with and without tags and subsequently purified.

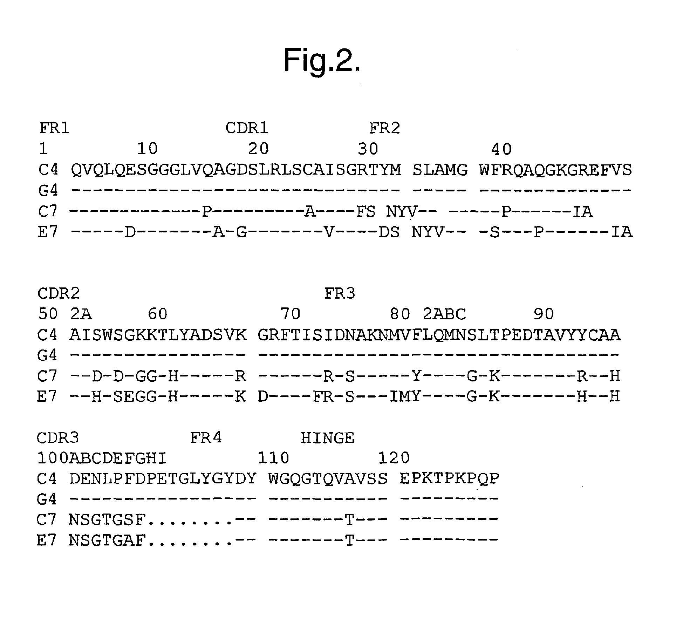

Sequence analysis revealed that the VHHs C4 and G4, both derived from the α-Mab library, were identical. The two V_{HH}S derived from the α-Fc library, C7 and E7, were very similar, but showed some differences in sequence (Figure 2).

Mouse isotype IgG specific VHH fragments were obtained with the counter selection method described in Example 3. The mouse IgG molecules can be divided into four subclasses based on the differences in the constant regions: IgG1, 2a, 2b and 3. To prevent the selection of antibody fragments for epitopes in the variable region, which are very different between the antibodies used in the selection, the α-Fc library was used for the selection of mouse IgG-isoype specific VHHs. To increase the efficiency of the selection of subclass-specific VHH, the selection of cross-reactive VHHs was reduced by the addition a twenty times excess of IgGs different from the IgG-subclass that was selected with (Table 6).

**Table 6**

| Selection of IgG-subclass specific VHH's. | | |
|---|---|---|
| In the selection α-RR6 (IgG1), α-TAG (IgG2a) and α-Muc (IgG3) were used | | |
| Library | Round 1 (5 nM) (counter-selection (0.1mM) ) (% positive in ELISA) | Round 2 (5nM) (counter-selection (0.1mM)) (% positive in ELISA) |
| α-Fc | 1A. IgG1 (IgG2a + IgG3) (42) | 1B. IgG1 (-) |
| | | 1C. IgG1 (IgG2a + IgG3) (33) |
| | 2A. IgG2a (IgG1 + IgG3) (50) | 2B. IgG2a (-) |
| | | 2C. IgG2a (IgG1 + IgG3) (50) |
| | 3A. IgG3 (IgG1 + IgG2a) (33) | 3B. IgG3 (-) |
| | | 3C. IgG3 (IgG1 + IgG2a) (38) |

VHHs specifically binding one immunoglobulin subclass were found even after one single round of selection. VHH fragments of both the first and the second round and giving positive responses in the ELISAs were analysed on BIAcore as performed previously with the V_{HH}S selected for cross-reacivity. The V_{HH}S were allowed to bind to a chip that was coated with IgG1 (α-Traseolide), IgG2a (α-TAG) and IgG3 (α-Muc).

The results of the BIAcore analysis disagree in some cases with the results obtained by ELISA screening. All V_{HH}S were selected for positive binding to antigen, but not every V_{HH}S showed this interaction on BIAcore. None of the α-IgG3 V_{HH}S bound to the IgG3 immobilised on the BIAcore chip.
Per subclass two V_{HH}S were selected for further analysis. To increase the chance of obtaining two different V_{HH}^{S} per subclass, a V_{HH} that showed a high response on BIAcore and a V_{HH} that showed a low response were chosen from both the IgG1 and the IgG2a specific fragments. The differences in amino acid composition could account for the differences in behaviour of these particular V_{HH}S. As a result of the fact that none of the analysed α-IgG3 V_{HH}S reacted positively on BIAcore, two ELISA-positive V_{HH}S were chosen randomly. The selected V_{HH}S were produced with tags and subsequently purified.

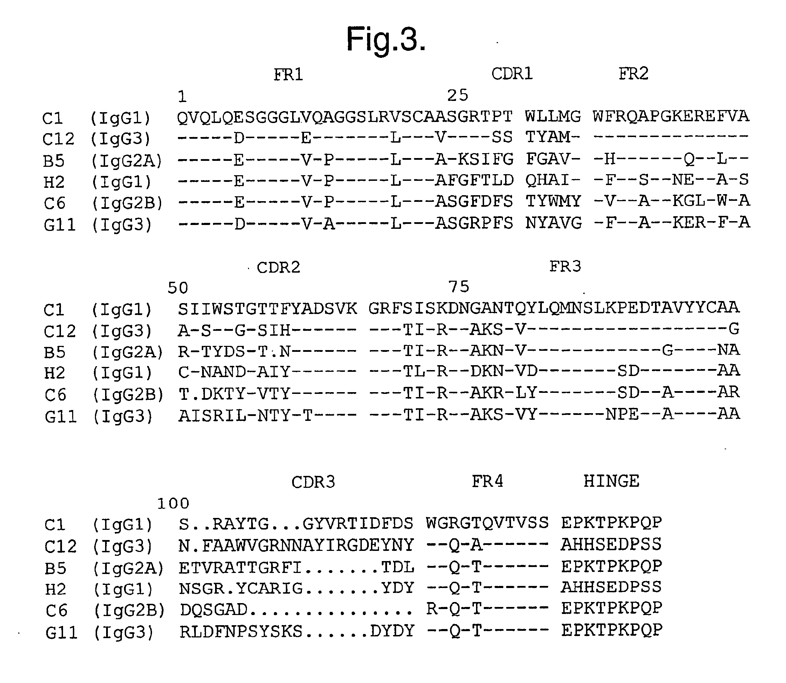

The sequences of the six purified V_{HH}S were determined and from this analysis it appeared that V_{HH} C12 and H2 are short hinge antibodies, while the other V_{HH}S are long-hinged (van der Linden et *al.,* 2000). The amino acid sequences are shown in Figure 3. The V_{HH}S show many variation in the CDRs, indicating that the chosen strategy of selecting V_{HH}S on behavioural properties was successful.

### 8.2.2 Antibody arraying

Mouse IgG (monoclonal antibodies of different isotypes) were labelled with Cy3 or Cy5 reactive dye (Amersham Pharmacia Biotech) according to the manufacturer's protocol. VHHs and proteins that served as negative or positive control were diluted to 4 different concentrations (100, 50, 25 and 12,5 µg/ml) in PBS and in 1% BSA (bovine serum albumin). A 96-wells microtiter plate was filled with 50 µl of each solution. Four empty wells were filled with PBS and four were filled with 1% BSA to serve as negative controls.

A GMS 417 arrayer (Genetic MicroSystems, Westburg BV) printed these protein solutions onto amino silane coated microscope glass slides. The slides were blocked for 1 hour in a solution of 3% Marvel in PBS, which had first been spun to remove particulate matter (10' at 2500 rpm). The slides were then washed in PBST to remove small deposits of Marvel. Each array was incubated for 1 hour with 20 µl solution of antigen (100 µg/ml), under a cover slip. The arrays were washed in PBS and then air-dried. The arrays were read by a GMS 418 Array Scanner, and the resulting data were subsequently analysed by specialised software developed by Genetic MicroSystems.

To explore the suitability of V_{HH}S for array applications, a number of identical copies of an α-mouse IgG antibody array was constructed using four selected IgG-subclass specific V_{HH}S (B5, C1, G11 and H2) and the IgG1, IgG2a and IgG3 cross-reactive V_{HH} E7. A list of V_{HH}S and proteins that served as positive and negative controls is presented in Table 7.

**Table 7**

| Components of α-mouse IgG antibody array | |
|---|---|
| Name | Function |
| B5 | α-IgG2a |
| C1 | α-IgG1 |
| G11 | α-IgG3 |
| H2 | α-IgG3 |
| E7 | α-IgG1 |
| IgG2a-Cy5 | Positive control on red fluorescence |
| IgG2a-Cy3 | Positive control on green fluorescence |
| IgG2a-Cy3/Cy5 | Control on relative intensities of red and green fluorescence |
| α-AFP | Negative control |
| α-GST | Negative control |
| Protein A | Positive control on antigen concentration |

Each α-mouse antibody array was probed with different, fluorescently labelled antigen(s). See Table 8 below.

**Table 8**

| Antigens applied to the α-mouse antibody arrays. | |
|---|---|
| The antigens shown in italics were labelled with a Cy3 fluorescent tag and the antigens marked with an *) with Cy5. | |
| Array nr. | Antigen (s) |
| 1 | *IgG1*/*CY3* |
| 2 | *IgG2a* / *CY3* |
| 3 | *IgG3* / *CY3* |
| 4 | α-*MYC* / *CY3* |
| 5 | *) IgGI / *) CY5 |
| 6 | *) IgG2a / CY5 |
| 7 | *) IgG3 / CY5 |
| 8 | *) α-MYC / CY5 |
| 9 | *) Mouse Fc / CY5 |
| 10 | *) Mouse serum / CY5 |
| 11 | *) IgGI, 2a, 3 / *) CY5 |
| 12 | *) Mouse Fc / CY5 + *IgG1, 2a, 3* / *CY3* |
| 13 | *) Mouse serum / CY5+ *IgGl, 2a, 3* / *CY3* |
| 14 | *) IgG1, 2a, 3 / CY5+ *IgG1, 2a,* 3 / *CY3* |
| 15 | Blanc CY3 |

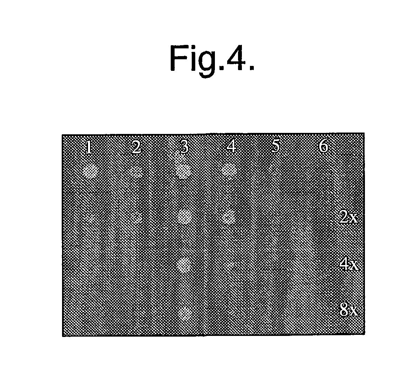

After scanning, different positive signals were obtained from the different arrays (see Figure 4).

### 8.2.3 Removal of abundant proteins from samples for array analysis by affinity chromatography with VHH fragments

Protein extracts from cells were prepared with physical methods as described in Examples 1, 5.1, 6.1 and 7.1. The biochemical methods are based on affinity purification of these proteins with the variable domain of antibodies of Camelidae bound to a solid support.

As a typical example the removal of albumin and IgG from mouse serum by affinity chromatography with VHH is demonstrated. Antibody fragments recognizing mouse serum albumin were selected from the naive library. The VHH encoding gene fragments were recloned in the *E. coli* expression vector pUR5850, which is identical to the phagemid vector pUR5071, but lacking the gene 3 needed for display on the phage vector. The VHHs was expressed with carboxyterminal c-myc- and His6-tag, purified with TALON (Clontech) and used for covalent coupling to a support.

To prepare a chromatography support with covalently coupled antibody, 12 mg of VHH in 21 ml buffer (0.1 M NaHCO₃, 0.5 M NaCl pH 8.3) was immobilized to 2 g of CNBr-activated Sepharose 4B (Amersham Biotech) according to the manufacturer's protocol. The column material (final volume 7 ml) was packed in a XK 16/20 column (Pharmacia). After treatment with blocking buffer (0.1 M Tris, 0.5 M NaCl pH 8.0) the affinity support was washed in an alternating fashion with Phosphate Buffered Saline (PBS: 10 mM Na₂HPO₄, 150 mM NaCl pH 7.4) as equilibration buffer and pH adjusted PBS (pH 2.1) as elution buffer.

A sample of mouse serum (50 µl) was loaded with a flow rate of 0.5 ml/min; if necessary, the sample was recycled in a closed loop. The non-bound material was recovered and analyzed on a coomassie stained 1D- or 2D-gel and compared with an untreated serum sample. After a single-round of depletion on the VHH column more than 99% of the serum albumin was removed.

The albumin depleted serum sample was loaded on an affinity matrix containing the anti-mouse IgG (VHH C4 (see Example 8.2.1) as was performed before (see above) . The non-bound fraction was analyzed on coomassie stained gels and revealed that more than 90% of the serum IgG was removed. On 2D-gel spots could be identified after staining, which were not visible before the depletion steps, showing that the resolution is improved.

The albumin depleted serum sample was used for labelling with Cy3 or Cy4 and subsequently analyzed on the antibody array (see Example 8.2.2). A much better signal to noise ratio and improved sensitivity was obtained compared with a non-depleted serum sample.

### EXAMPLE 9 - Evaluation of the quality and diversity of the library of single chain domain antibodies by selection with various antigens

The quality of the library was established by a selection with different kind of antigens. First of all, a large number of protein antigens was tested. A panel of human proteins, including the interleukins IL4, IL6 and IL7, immunoglobulins, the gene product PKD1 involved in human polycystic kidney disease 1, gene and human serum albumin yielded specific single-domain antibody fragments and a series of anti-idiotypic V_{H}H-fragments was selected against a humanized anti-hCD4 antibody of human origin, which allowed the quantification of the anti-CD4-antibody in human serum up to a concentration of 0.5 nM. A second group consisted of proteins from eukaryotic non-human origin, including IgG from mouse and pig. A third group consisted of different prokaryotic organisms, including *Bacillus subtilis, Pseudomonas aeruginosa, Mycobacterium paratuber-culosis, Klebsiella pneumoniae* and the BabA surface antigen of *Helicobacter pylori.* Again, the obtained fragments could be used for the specific detection of the bacteria, even in the presence of high concentrations of detergents. A fourth group consisted of proteins from bacteriophages and viruses, including lysin from *Lactococcus lactis* phage p2 and the envelop of salmon pancreas disease virus (SPDV). A fifth group consisted of receptor molecules, including the extracellular domain of the human glutamate receptor (mGLU4R) and an extracellular loop of the drug pump pdr12 of yeast. A sixth group consisted consisted of proteins, and post-translationally modifications thereof, from yeasts.

Secondly, the library contained anti-self antibodies. As target a V_{H}H-fragment without tags (myc and His6) delivered specific antibodies, which bind these and related molecules probably by recognizing an epitope in the free C-terminal end, which is encoded by the FR4-region.

Thirdly, a group of haptens was used for selection, which in general does not elicit heavy-chain antibodies upon immunisation of llamas or camels. The haptens tested were the hormone estrone 3 glucuronide (E3G), yielding cross-reactive fragments against estradiol, and also estrone-specific V_{H}H's. Furthermore, antibody fragments were selected against 5-(2',3',5',6'-tetrachloro-4'-oxyphenyl) valeric acid and the related molecule 5-(2',3',5',6'-tetrachloro-4'-methoxyphenyl) valeric acid, the azodye Reactive Red 6 and against phytoestrogene.

## Claims

1. A protein array which comprises a plurality of heavy-chain variable domains derived from an immunoglobulin that is naturally devoid of light chains (VHH domain) obtainable from Camelidae, and wherein the heavy-chain variable domains are derivatised with a chemical group through which the heavy-chain variable domains are coupled to the array, or wherein the heavy-chain variable domains are coupled to the array through a peptide extension,
with the provision that the heavy-chain variable domains are not covalently coupled to the array by a polypeptide segment which is capable of adopting a folded structure separate from the folded structure of the remainder of the heavy-chain variable domains, the polypeptide segment containing at least one cross-linkable residue for covalent attachment to a solid surface and wherein the polypeptide segment has the generic structure AZB wherein A and B are regions of peptide sequence that can come together to form a structure, and Z is a region of peptide sequence able to form a turn.

2. A protein array according to claim 1 comprising heavy-chain variable domains from a library comprising cloned DNA sequences encoding heavy-chain variable domains, where clones are derived from an unimmunised animal of the genus Camelidae.

3. A protein array according to claim 1 or 2, further comprising:
(a) a solid surface;
(b) a plurality heavy-chain variable domains immobilised at distinct positions on the solid surface, wherein each heavy-chain variable domain is capable of binding to a specific protein antigen.

4. A protein array according to any one of claims 1 to 3, wherein the heavy-chain variable domains have been derived by selection from a library using the phage or lower eukaryote display method.

5. A protein array according to any one of claims 1 - 4, wherein the heavy-chain variable domains have been immobilized by chemical binding via N- or C- terminal peptide extensions of the heavy-chain variable domains.

6. A protein array according to any one of the preceding claims, wherein the heavy-chain variable domains are biotinylated and are coupled to the array with a surface derivatised with streptavidin.

## Patentansprüche

1. Proteinarray, das mehrere variable Domänen von schweren Ketten aufweist, die von einem Immunglobulin stammen, das von Natur aus ohne leichte Ketten ist (VHH-Domäne), das von Kamelen erhalten werden kann, und wobei die variablen Domänen der schweren Ketten ein Derivat mit einer chemischen Gruppe gebildet haben, durch die die variablen Domänen der schweren Ketten an dieses Array gebunden sind, oder wobei die variablen Domänen der schweren Ketten durch Peptidverlängerung an dieses Array gebunden sind,
mit der Maßgabe, daß die variablen Domänen der schweren Ketten nicht durch ein Polypeptidsegment kovalent an das Array gebunden sind, das getrennt von der gefalteten Struktur der übrigen variablen Domänen der schweren Ketten eine gefaltete Struktur annehmen kann, wobei das Polypeptidsegment mindestens einen vernetzbaren Rest für eine kovalente Bindung an eine feste Oberfläche enthält und wobei das Polypeptidsegment die generische Struktur AZB aufweist, wobei A und B Regionen der Peptidsequenz sind, die zusammenkommen können, so daß eine Struktur entsteht, und wobei Z eine Region der Peptidsequenz ist, die eine Schleife bilden kann.

2. Proteinarray nach Anspruch 1,
das variable Domänen der schweren Ketten aus einer Bank aufweist, die geklonte DNA-Sequenzen aufweist, die variable Domänen der schweren Ketten codieren, wobei die Klone von einem nichtimmunisierten Tier der Gattung Camelidae stammen.

3. Proteinarray nach Anspruch 1 oder 2,
das ferner folgendes aufweist:
(a) eine feste Oberfläche;
(b) mehrere variable Domänen der schweren Ketten, die an unterschiedlichen Positionen auf der festen Oberfläche immobilisiert sind, wobei jede variable Domäne der schweren Ketten an ein bestimmtes Proteinantigen binden kann.

4. Proteinarray nach einem der Ansprüche 1 bis 3,
wobei die variablen Domänen der schweren Ketten durch Auswahl aus einer Bank unter Anwendung des Phagendisplay-Verfahrens oder des Displayverfahrens mit niederen Eukaryoten abgeleitet sind.

5. Proteinarray nach einem der Ansprüche 1 bis 4,
wobei die variablen Domänen der schweren Ketten durch chemisches Binden über N- oder C-terminale Peptidverlängerungen der variablen Domänen der schweren Ketten immobilisiert sind.

6. Proteinarray nach einem der vorstehenden Ansprüche,
wobei die variablen Domänen der schweren Ketten biotinyliert und an das Array mit einer Oberfläche gebunden sind, die ein Derivat mit Streptavidin gebildet hat.

## Revendications

1. Matrice de protéines comprenant une pluralité de domaines variables de chaînes lourdes issus d'une immunoglobuline qui est naturellement dépourvue de chaînes légères (domaine VHH), pouvant être obtenue à partir de camélidés, et dans laquelle les domaines variables de chaînes lourdes sont dérivatisés avec un groupe chimique grâce auquel les domaines variables de chaînes lourdes sont couplés à la matrice, ou dans laquelle les domaines variables de chaînes lourdes sont couplés à la matrice par l'intermédiaire d'une extension peptidique,
à condition que les domaines variables de chaînes lourdes ne soient pas couplés par liaison covalente à la matrice par un segment polypeptidique qui est capable d'adopter une structure repliée distinctes de la structure repliée du reste des domaines variables de chaînes lourdes, le segment polypeptidique contenant au moins un résidu réticulable permettant une fixation covalente à une surface solide et le segment polypeptidique possédant la structure générique AZB où A et B sont des régions de séquences peptidiques qui peuvent s'assembler pour former une structure et Z est une région de séquence peptidique capable de former un coude.

2. Matrice de protéines selon la revendication 1, comprenant des domaines variables de chaînes lourdes provenant d'une banque contenant des séquences d'ADN cloné codant des domaines variables de chaînes lourdes, les clones provenant d'un animal non immunisé du genre camélidé.

3. Matrice de protéines selon la revendication 1 ou 2, comprenant en outre :
(a) une surface solide ;
(b) une pluralité de domaines variables de chaînes lourdes immobilisés à des positions distinctes sur la surface solide, chaque domaine variable de chaîne lourde étant capable de se lier à un antigène protéique spécifique.

4. Matrice de protéines selon l'une quelconque des revendications 1 à 3, dans laquelle les domaines variables de chaînes lourdes ont été dérivés par sélection dans une banque au moyen d'un procédé d'exposition sur phage ou sur un eucaryote inférieur.

5. Matrice de protéines selon l'une quelconque des revendications 1 à 4, dans laquelle les domaines variables de chaînes lourdes ont été immobilisés par liaison chimique par l'intermédiaire d'extensions peptidiques N- ou C-terminales des domaines variables de chaînes lourdes.

6. Matrice de protéines selon l'une quelconque des revendications précédentes, dans laquelle les domaines variables de chaînes lourdes sont biotinylés et sont couplés à la matrice ayant une surface dérivatisée avec de la streptavidine.


### SEQUENCE LISTING

<110> UNILEVER N.V./Unilever PLC
<120> PROTEIN ARRAYS
<130> T3083(V)
<160> 4
<170> PatentIn Ver. 2.1
<210> 1
   <211> 23
   <212> DNA
   <213> Artificial Sequence
<220>
   <223> Description of Artificial Sequence:PCR-primer
<400> 1
   gaggtbcarc tgcaggastc ygg 23
<210> 2
   <211> 53
   <212> DNA
   <213> Artificial Sequence
<220>
   <223> Description of Artificial Sequence:PCR-primer
<400> 2
   aacagttaag cttccgcttg cggccgcgga gctggggtct tcgctgtggt gcg 53
<210> 3
   <211> 53
   <212> DNA
   <213> Artificial Sequence
<220>
   <223> Description of Artificial. Sequence:PCR-primer
<400> 3
   aacagttaag cttccgcttg cggccgctgg ttgtggtttt ggtgtcttgg gtt 53
<210> 4
   <211> 32
   <212> PRT
   <213> Artificial Sequence
<220>
<223> Description of Artificial Sequence:multi-tag fused to VHH carboxy terminus
<400> 4
   Glu Gln Lys Leu Ile Ser Glu Glu Asp Leu Asn Gly Ala Ala Leu Arg
     1               5                  10                  15
   Ser Ile Phe Glu Ala Gln Lys Met Glu Trp His His His His His His
                20                  25                  30